(19) **Europäisches Patentamt / European Patent Office / Office européen des brevets**

(11) **EP 4 772 160 A1**

(12) **EUROPEAN PATENT APPLICATION**
published in accordance with Art. 153(4) EPC

(43) Date of publication:
**08.07.2026 Bulletin 2026/28**

(21) Application number: **24859582.9**

(22) Date of filing: **22.08.2024**

(51) International Patent Classification (IPC):
*A61K 8/25* (2006.01)          *A61Q 1/00* (2006.01)
*A61Q 1/02* (2006.01)          *A61Q 1/04* (2006.01)
*A61Q 1/10* (2006.01)          *A61Q 1/12* (2006.01)
*A61Q 3/02* (2006.01)          *A61Q 19/00* (2006.01)
*C09C 3/06* (2006.01)          *C09C 3/12* (2006.01)

(52) Cooperative Patent Classification (CPC):
A61K 8/25; A61Q 1/00; A61Q 1/02; A61Q 1/04;
A61Q 1/10; A61Q 1/12; A61Q 3/02; A61Q 19/00;
C09C 3/06; C09C 3/12

(86) International application number:
**PCT/JP2024/029725**

(87) International publication number:
**WO 2025/047549 (06.03.2025 Gazette 2025/10)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **28.08.2023 JP 2023138448**

(71) Applicants:
- **SHIN-ETSU CHEMICAL CO., LTD.**
  **Tokyo 100-0005 (JP)**
- **Nissin Chemical Industry Co., Ltd.**
  **Fukui 915-0802 (JP)**

(72) Inventors:
- **TANAKA Mitsuru**
  **Tokyo 100-0005 (JP)**
- **INABA Ryuichi**
  **Tokyo 100-0005 (JP)**

- **INOKUCHI Yoshinori**
  **Annaka-shi, Gunma 379-0224 (JP)**
- **MATSUMURA Kazuyuki**
  **Annaka-shi, Gunma 379-0224 (JP)**
- **TAKAHASHI Shohei**
  **Annaka-shi, Gunma 379-0195 (JP)**
- **KOBAYASHI Yuji**
  **Annaka-shi, Gunma 379-0195 (JP)**
- **INUKAI Tetsuya**
  **Annaka-shi, Gunma 379-0195 (JP)**
- **CHIBA Shunsuke**
  **Echizen-shi, Fukui 915-0802 (JP)**
- **WATANABE Kentaro**
  **Echizen-shi, Fukui 915-0802 (JP)**
- **OKUDA Harukazu**
  **Echizen-shi, Fukui 915-0802 (JP)**

(74) Representative: **Schicker, Silvia**
**Wuesthoff & Wuesthoff**
**Patentanwälte und Rechtsanwalt PartG mbB**
**Schweigerstraße 2**
**81541 München (DE)**

(54) **COMPOSITE POWDER AND COSMETIC PREPARATION**

(57)    The present invention is a composite powder including a pearl pigment and spherical silica fixed thereon. This provides a composite powder and a cosmetic that provide a natural, transparent finish while correcting skin irregularities, etc. and further provide the effect of brightening the skin while suppressing unnatural luster, and are excellent in in-use feeling such as smoothness.

**Description**

TECHNICAL FIELD

The present invention relates to a composite powder and a cosmetic.

BACKGROUND ART

One of the purposes of cosmetics is to make skin look smooth and beautiful by concealing morphological problems such as wrinkles, pores, and rough texture, as well as color tone problems such as blemishes and freckles. In recent years, emphasis has been placed on a natural, unartificial finish (bare skin feel). In addition, a natural cosmetic finish is defined as one that is free of unnatural gloss (luster), is excellent in uniformity of cosmetic film, and is highly transparent. Titanium dioxide pigments, which have a high refractive index and concealing properties, have conventionally been used in cosmetics, but this has resulted in unnatural finish without gloss, and being far from the impression of translucent skin.

Pearl pigments, which are platelet-shaped powders coated with metal oxides, are commonly known. Pearl pigments utilize light interference to enable a wide range of distinctive colors. Patent Document 1 discloses a method for blending fine pearl pigments with spherical resin powder in optimal amounts and ratios. However, the optical properties of the pearl pigments and spherical resin powder cancel each other out, resulting in an unsatisfactory finish.

Meanwhile, many novel materials and technologies have been proposed in order to achieve a natural-looking finish. Cosmetics containing various diffuse reflective powders are known as a method for concealing morphological problems, in particular. Patent Document 2 discloses a flaky fine powder in which the surface of a flaky material such as natural mica is coated with spherical silica microparticles. This flaky fine powder uses spherical microparticles that have a high surface diffuse scattering effect for light, and thus when this flaky fine powder is used as a cosmetic ingredient, it is said to have the following technical effects: "It can suppress the gloss at the corners of the mica substrate and produce a so-called soft focus effect, such as making fine wrinkles less noticeable. It can also provide cosmetics with significantly improved smoothness and feel during use". However, the flaky fine powder has such excellent smoothness, and thus cosmetics containing this flaky fine powder are problematic in that they tend to crease easily upon application, making it difficult to apply evenly, and resulting in a lack of a natural finish.

Patent Document 3 discloses a composite powder in which a silicone elastomer adheres to a core powder such as an inorganic powder, organic powder, or inorganic-organic composite powder. While the light diffusion effect has been satisfactory for correcting skin problems, the silicone elastomer has provided an excellent soft in-use feeling, leaving room for improvement in terms of smoothness. In addition, in the composite powders described in Patent Documents 2 and 3, the particles adhering to the base particles do not exhibit a monodisperse particle size distribution, and thus further research is needed to correct color tone problems such as blemishes and freckles on the skin and create a more natural-looking skin.

CITATION LIST

PATENT LITERATURE

Patent Document 1: JP2005-097218A
Patent Document 2: JP2784261B2
Patent Document 3: JP5359593B2

SUMMARY OF INVENTION

TECHNICAL PROBLEM

The present invention has been made in consideration of the above circumstances, and an object thereof is to provide a composite powder and a cosmetic that provide a natural, transparent finish while correcting skin irregularities, etc. and further provide the effect of brightening the skin while suppressing unnatural luster, and that also have excellent in-use feeling, such as smoothness.

SOLUTION TO PROBLEM

In order to solve the above problems, the present invention provides a composite powder comprising a pearl pigment and spherical silica fixed thereon.

This type of composite powder is a composite powder that provides a natural, transparent finish while correcting skin irregularities, etc. and further provides the effect of brightening the skin while suppressing unnatural luster, and that also has excellent in-use feeling, such as smoothness.

In addition, in the present invention, the spherical silica is preferably in an amount of 10 to 50 parts by mass based on 100 parts by mass of the pearl pigment.

Such a composite powder provides the structural color and light scattering effect of the coated spherical silica and the interference color effect of the pearl pigment, without the gloss of the pearl pigment.

In addition, in the present invention, the average particle size of the spherical silica is preferably in the range of 100 to 500 nm.

Such spherical silica exhibits excellent adhesion of the spherical silica particles to the surface of the pearl pigment, allowing a uniform coating of the pearl pigment.

In addition, in the present invention, it is preferable that a particle size distribution of the spherical silica is monodisperse.

Such a composite powder does not have variation in particle size, and the structural color exhibited by the spherical silica does not weaken.

In addition, in the present invention, it is preferable that a hue angle difference $\Delta h$ between an interference color due to reflection of the pearl pigment and a structural color due to reflection of the spherical silica is in the range of 0 to 90.

Such a composite powder can further enhance the effects of the present invention.

In addition, in the present invention, it is preferable that the spherical silica is chemically fixed on the pearl pigment with a binder.

Such a composite powder prevents the spherical silica fixed on the pearl pigment from peeling off due to mechanical stress during cosmetic manufacturing, allowing the powder to remain effective after the cosmetic manufacturing process.

In this case, it is preferable that the binder is a resin or silica.

Such a composite powder causes the spherical silica to be fixed on the surface of the pearl pigment, making it less likely to fall off from the surface of the pearl pigment, allowing a more favorable in-use feeling to be imparted.

In addition, it is preferable that the binder is in the range of 5 to 50 parts by mass based on 100 parts by mass of the total amount of the pearl pigment and the spherical silica.

Such a composite powder causes the spherical silica to be sufficiently fixed on the surface of the pearl pigment, the product does not feel hard to the touch, and the powder does not aggregate together.

In addition, it is preferable that the composite powder is subjected to a hydrophobic surface treatment.

Such a composite powder can impart or improve water repellency and improve dispersibility in oil agents.

In addition, the present invention provides a cosmetic comprising the composite powder described above.

Such a cosmetic is a cosmetic that provides a natural, transparent finish while correcting skin irregularities, etc. and further provides the effect of brightening the skin while suppressing unnatural luster, and that also has excellent in-use feeling, such as smoothness.

In this case, it is preferable that the cosmetic is a skin care cosmetic.

In addition, it is preferable that the cosmetic is a makeup cosmetic.

The cosmetic of the present invention can be used for such purposes.

In addition, the present invention provides a composite powder, comprising a pearl pigment and a spherical powder fixed thereon.

Such a composite powder is a composite powder that provides a natural, transparent finish while correcting skin irregularities, etc. and further provides the effect of brightening the skin while suppressing unnatural luster, and that also has excellent in-use feeling, such as smoothness.

ADVANTAGEOUS EFFECTS OF INVENTION

The present invention can provide a composite powder that provides a natural, transparent finish while correcting skin irregularities, etc. and further provides the effect of brightening the skin while suppressing unnatural luster, and that also has excellent in-use feeling, such as smoothness, and a cosmetic including the composite powder.

DESCRIPTION OF EMBODIMENTS

As described above, there has been required the development of a composite powder and a cosmetic that provide a natural, transparent finish while correcting skin irregularities, etc. and further provide the effect of brightening the skin while suppressing unnatural luster, and that also have excellent in-use feeling, such as smoothness.

As a result of extensive research into solving the above problems, the present inventors have found that a composite powder with spherical silica or spherical powder fixed on a pearl pigment provides a natural, transparent finish while correcting skin irregularities, etc. and further provides the effect of brightening the skin while suppressing unnatural luster, and that also has excellent in-use feeling, such as smoothness, and the present invention has been completed.

That is, the present invention is a composite powder, including a pearl pigment and spherical silica fixed thereon.

In addition, the present invention is a composite powder, including a pearl pigment and a spherical powder fixed thereon.

The present invention is described in detail below. In addition, in this invention, ingredient names may be written as Cosmetic Ingredient Labeling Name or International Nomenclature of Cosmetic Ingredients (INCI). When Cosmetic Ingredient Labeling Name and INCI correspond, the English name may be omitted.

[Composite Powder]

The composite powder of the present invention is a composite powder, including a pearl pigment and spherical silica or a spherical powder fixed thereon. Each ingredient included in the composite powder of the present invention will be explained in detail below.

[Pearl pigment]

The pearl pigment in the present invention is a pearl pigment obtained by forming a coating layer primarily composed of titanium dioxide or iron oxide on a plate-shaped powder surface. The pearl pigment of the present invention is a pearl pigment in which the surface of a plate-shaped powder such as mica, talc, sericite, synthetic fluorphlogopite, or glass flake is uniformly coated with a colorant such as titanium dioxide, iron oxide, silicon oxide, Prussian blue, chromium oxide, tin oxide, chromium hydroxide, gold, silver, carmine, or an organic pigment.

The pearl pigment in the present invention preferably has an average particle size, as determined by microscopic observation, of typically 1 $\mu$m to 300 $\mu$m, more preferably 2 $\mu$m to 200 $\mu$m, still more preferably 5 $\mu$m to 100 $\mu$m, and particularly preferably 5 $\mu$m to 60 $\mu$m. The average particle size is the average value of the maximum length of the flaky powder. An average particle size of 1 $\mu$m or more results in finish with excellent transparency, while an average particle size of 300 $\mu$m or less results in an appropriate level of gloss.

Pearl pigments can be used without any particular restrictions as long as they are generally used in cosmetics.

Examples of commercially available pearl pigments include: Flamenco series (manufactured by DIC Corporation) such as Flamenco Superpearl, Flamenco Ultra Sparkle, Flamenco Red, Flamenco Blue, Flamenco Green, Flamenco Super Red, Flamenco Summit Gold, Flamenco Sparkle Orange, Flamenco Satin Red, Flamenco Satin Violet, Flamenco Satin Blue, and Flamenco Twilight Red; Timiron series (manufactured by Merck KGaA) such as Timiron Super Violet, Timiron Supersilk MP-1005, Timiron Supersheen MP-1001, Timiron Starluster MP-115, Timiron SynBeam Red, Timiron Silk Blue, Timiron Splendit Blue, and Timiron Starlight Blue; Metashine series (manufactured by Nippon Sheet Glass Co., Ltd.) such as Metashine MT1040, MT1080, MT1120, and MC2080PS; and Helios series (manufactured by Topy Industries, Ltd.) such as Helios R10, R20, and R100. Colorona series (Merck kGaA), Cosmicolor series (Toyo Aluminium K.K.), Timica series, Cloisonne series, INTENZA series, SunPURO series (DIC Corporation), Twinclepearl series (Nihon Koken Kogyo Co., Ltd.), FANTASPEARL series (Nihon Koken Kogyo Co., Ltd.), KOBOPEARL series (Kobo Products, Inc.), Glare series (CQV Co., Ltd.), and the like can also be suitably used.

[Spherical Silica]

The spherical silica used in the composite powder of the present invention will be described. Silica is generally broadly divided into wet-process silica and dry-process silica. The resulting silica comes in a variety of shapes, including spherical, plate-shaped, spindle-shaped, cubic, and irregular, but spherical silica is used in the present invention.

Non-spherical particles used in cosmetics does not act as a type of ball bearing during application of cosmetics, failing to achieve the effect of dramatically improving the smoothness of the composite powder. In addition, it is difficult to achieve the effect of effectively scattering light on the surface of the cosmetic coating film and concealing skin morphological problems related to light reflection (soft focus effect).

In the context of the spherical silica of the present invention, "spherical" refers not only to perfect spheres but also to slightly distorted spheres. The shape of such particles can be evaluated in terms of the circularity of the particle when projected two-dimensionally. The circularity is defined as (perimeter of a circle equal to the particle area) / (particle perimeter). Herein, "spherical" in the present invention particularly refers to an average circularity in the range of 0.8 to 1. Preferably, the average circularity is 0.85 to 1, and more preferably 0.90 to 1. Also, the circularity can be measured by analyzing images obtained with an electron microscope or the like.

The spherical silica used in the present invention may be hydrophilic spherical silica, or hydrophobic spherical silica obtained by subjecting the surface of spherical silica to a hydrophobic treatment.

In the case of hydrophilic spherical silica, dispersion in an aqueous ingredient is preferable, while in the case of hydrophobic spherical silica, dispersion in an oily ingredient is preferable.

As spherical silica, any of the following types, as defined by Cosmetic Ingredient Labeling Name, can be suitably used: silica, hydrated silica, silica dimethicone silylate, dimethyl silylated silica, and the like.

It is known that silica exhibits structural color when nanoparticleized. In addition, the structural color exhibited varies depending on the particle size of the resulting silica. To effectively exhibit any structural color, it is preferable for the shape and particle size to be uniform. For this reason, it is preferable that the spherical silica used in the composite powder of the present invention has a high degree of monodispersity.

The spherical silica is preferably in an amount of 10 to 50 parts by mass, more preferably 10 to 40 parts by mass, and still more preferably 10 to 35 parts by mass based on 100 parts by mass of the pearl pigment.

When the spherical silica is in an amount of 10 parts by mass or more based on 100 parts by mass of the pearl pigment, the structural color and light-scattering effect of the coated spherical silica are obtained, and the gloss of the pearl pigment is not exhibited. In addition, when the spherical silica is in an amount of 50 parts by mass or less, the light-scattering effect and interference color effect of the pearl pigment are obtained.

The average particle size of the spherical silica is preferably 100 to 500 nm. When the average particle size of the spherical silica is in the range of 100 nm or more, the structural color and light-scattering effect of the coated spherical silica are obtained, and the effect of correcting the irregularities of the skin is obtained. In addition, the contact area with the skin is smaller, resulting in excellent smoothness. When the average particle size is 500 nm or less, the spherical silica has excellent adhesion to the surface of the pearl pigment, allowing a uniform coating on the pearl pigment, and is thus effective in covering skin color defects such as dullness, spots, and freckles.

The above average particle size indicates the volume median diameter (D50) value. The volume median diameter (D50) is the particle size corresponding to 50% of the cumulative distribution when the volume particle size distribution is expressed as a cumulative distribution. The volume median diameter can be measured, for example, using a laser diffraction/scattering particle size distribution analyzer.

The particle size distribution of the spherical silica may be a mixture of polydispersed with low monodispersity or monodispersed particles, but monodispersion is more preferable to enhance the effect of structural color. Specifically, the volume particle size distribution D90/D10 value of the spherical silica is preferably 3 or less, and more preferably 2.9 or less. In a powder particle size distribution, the particle size at which the cumulative volume reaches 10% from the smaller particle size side is referred to as D10, and the particle size at which the cumulative volume reaches 90% from the smaller particle size side is referred to as D90. A D90/D10 value of 3 or less, more preferably 2.9 or less, means that the particle size distribution is sharp (single) and the silica is monodispersed spherical.

When the D90/D10 value is 3 or less, the particle size distribution is sharp, the particle size does not vary, and the structural color exhibited by the spherical silica is not weaken.

The method for manufacturing spherical silica in the present invention is not particularly limited, and can be performed by any known method, such as the sol-gel method. The sol-gel method is a manufacturing method by the process of adding a tetrafunctional silane compound to a mixture of a hydrophilic organic solvent such as alcohol, water, and a basic substance, allowing the mixture to react to obtain a solvent dispersion including hydrophilic spherical silica microparticles, and then drying the solvent to obtain a powder.

A more detailed description of the method for manufacturing spherical silica in the present invention is provided below, but is not limited thereto.

[Hydrophilic spherical silica]

As an example of manufacturing hydrophilic spherical silica, 1,302.0 g of ethanol, 2.4 g of water, and 116.4 g of 28% aqueous ammonia are mixed, and the temperature of mixture is adjusted to 44°C, then, 656.4 g of tetraethoxysilane and a mixed solution of 127.2 g of water and 37.2 g of 28% aqueous ammonia are added dropwise over 3 hours, the mixture is stirred for another hour, and then heated to 100°C under normal pressure to replace the solvent with water, thereby obtaining monodispersed hydrophilic spherical silica.

[Hydrophobic spherical silica]

As an example of manufacturing hydrophobic spherical silica, 989.5 g of methanol, 135.5 g of water, and 66.5 g of 28% aqueous ammonia are mixed, the temperature of mixture is adjusted to 35°C, and 436.5 g of tetramethoxysilane is added dropwise over 6 hours while stirring, and then the mixture is stirred for 0.5 hours to continue hydrolysis, thereby obtaining a suspension of hydrophilic spherical silica microparticles. 4.4 g of methyltrimethoxysilane is added dropwise to this suspension at room temperature over 0.5 hours, and the mixture is continued to stir for 12 hours to hydrophobize the surface of the silica microparticles, thereby obtaining a hydrophobic spherical silica microparticle dispersion.

In addition, as another method, 217.0 g of ethanol, 2.3 g of water, and 27.5 g of 28% aqueous ammonia are mixed, and the temperature of mixture is adjusted to 60°C and 547.0 g of tetraethoxysilane and a mixed solution of 109.8 g of water and 27.5 g of 28% aqueous ammonia are added dropwise over 3 hours while stirring, and hydrolysis is continued for 0.5 hours to obtain a solvent dispersion of hydrophilic spherical sol-gel silica microparticles. 4.7 g of methyltriethoxysilane is added dropwise to this dispersion at room temperature over 0.5 hours, and then stirring is continued for 12 hours to obtain a dispersion of spherical silica in which a part of the silanol groups present on the surface of the silica microparticles have been hydrophobized. Further, 83.5 g of hexamethyldisilazane is added to this dispersion at room temperature, and then the mixture is heated to 50 to 60°C for 6 hours to trimethylsilylate the silanol groups. Then, the solvent is distilled off to obtain monodispersed hydrophobic spherical silica.

It is known that coloring occurs due to a fine periodic structure with a period on the order of the wavelength of visible light. This coloring is based on the diffraction and interference of light by the periodic structure and is generally referred to as structural color. The structural color has a unique gloss and is characterized by the fact that the color changes depending on the viewing angle. In addition,
the coloring is based on the diffraction and interference of light, and thus coloring materials that exhibit structural color, such as those that do not fade, are useful. In the present invention, when structural color is desired from spherical silica, it is preferable that the particles are uniform. In this case, monodispersion is preferable. The monodispersion refers to a narrow particle size distribution, and the coefficient of variation of particle size (a value of the standard deviation of particle size expressed as a percentage based on the average particle size) is preferably 10% or less, and more preferably 5% or less.

The composite powder of the present invention combines the structural colors of a pearl pigment and spherical silica, thereby exhibiting excellent coloring of interference color regardless of coloring by interference of light with any wavelength depending on the combination, but in order to effectively achieve the effects of the present invention (particularly the effect of improving color tone problems), it is preferable to do as follows.

Adjusting the average particle size and monodispersity is important to develop structural colors. When spherical silica is fixed on a plate-shaped powder, the average particle size and monodispersity of the spherical silica may change, but the above average particle size and monodispersity of the spherical silica refer to the state before the spherical silica is fixed on the plate-shaped powder.

A method for confirming the interference color due to reflection of the pearl pigment and the structural color due to reflection of spherical silica in the present invention will be described below.

The methods for measuring object color include the "visual comparison method" and the "physical colorimetric method". The "visual comparison method" is a method of evaluating a test item by visually comparing the test item with a color sample (such as a standard color chart conforming to JIS Z 8721). The "physical colorimetric method" is a method of measurement using a colorimetric device, and there are two main types.

1. A "direct tristimulus value reading method" in which a sample is measured with three sensors having spectral response characteristics substantially identical to those of the human eye, and the tristimulus values X, Y, and Z are directly measured.
2. A "spectrophotometric colorimetric method" in which light reflected from a sample is spectrally separated by a diffraction grating, etc., the reflectance is measured, and the tristimulus values X, Y, and Z are calculated by performing an integration calculation.

In the present invention, 2. spectrophotometric colorimetric method was adopted as the colorimetric method. A spectrophotometer was used as the colorimetric device. Examples of the spectrophotometer include the SE7700 manufactured by NIPPON DENSHOKU INDUSTRIES Co., Ltd. Many colorimetric devices can calculate the obtained tristimulus values X, Y, and Z and display them in various color systems. In the present invention, the L*a*b* color space was used as the color system. The L*a*b* color space was standardized by the International Commission on Illumination (CIE) in 1976, and is also adopted in Japan in JIS (JIS Z 8781-4). This index is a color space that is consistent with human perception, thus making it easy to intuitively grasp the color from the numerical values.

In addition, when the interference color due to reflection of a pearl pigment and the structural color due to reflection of spherical silica are observed, multiple scattering of visible light emphasizes the white color, thus making it difficult to clearly observe the structural color using the pearl pigment singly or silica singly. Therefore, spreading the pearl pigment or spherical silica over black artificial leather causes the scattering of visible light to be suppressed, and in a state of highlighting the structural color, color measurement was performed. Specifically, 0.01 g of a pearl pigment or spherical silica was applied to a 30 cm$^2$ piece of black artificial leather, and the color of the applied sample was measured with a spectrophotometer to calculate the L*a*b* values and quantify the structural color.

The interference color due to reflection of the pearl pigment and the structural color due to reflection of the spherical silica may be similar or different, but when they are similar, the optical properties of both the pearl pigment and the spherical silica act synergistically, thereby providing the cosmetic with a high-quality texture from complex optical properties.

A method for confirming the similarity between the interference color due to reflection of the pearl pigment and the structural color due to reflection of spherical silica in the present invention will be described below.

L*a*b* color space and L*C*h color space of CIE were used to evaluate structural colors. The L* value represents lightness, a* represents redness (+a* indicates a redder tone, -a* indicates a greener tone), and b* represents yellowness (+b* indicates a yellower tone, -b* indicates a bluer tone). In addition, the C* value represents saturation, and the h value represents the hue angle, calculated using the following formulae.

$$C^* = \sqrt{(a^*)^2 + (b^*)^2}$$

$$h = \tan^{-1}\left(\frac{b^*}{a^*}\right)$$

The lightness of the pearl pigment is defined as $L^*_P$, the a* value as $a^*_P$, the b* value as $b^*_P$, the saturation as $C^*_P$, and the hue angle as $h_P$, the lightness of the spherical silica is defined as $L^*_S$, the a* value as $a^*_S$, the b* value as $b^*_S$, the saturation as $C^*_S$, and the hue angle as $h_S$.

In addition, as an index to evaluate the similarity between the interference color due to reflection of the pearl pigment and the structural color due to reflection of the spherical silica, the hue angle difference $\Delta h$ between the pearl pigment and the spherical silica is defined as

$$\Delta h = \left| h_P - h_S \right|$$

L*a*b* color difference $\Delta E^*ab_{ps}$ between pearl pigment and monodispersed spherical silica is defined as

$$\Delta E^*ab_{ps} = \sqrt{\left(L^*_P - L^*_s\right)^2 + \left(a^*_P - a^*_s\right)^2 + \left(b^*_P - b^*_P\right)^2}$$

$$= \sqrt{\left(\Delta L^*_{ps}\right)^2 + \left(\Delta a^*_{ps}\right)^2 + \left(\Delta b^*_{ps}\right)^2}$$

Note that,

$$\Delta L^*_{ps} = L^*_p - L^*_s$$

$$\Delta a^*_{ps} = a^*_p - a^*_s$$

$$\Delta b^*_{ps} = b^*_p - b^*_s$$

The above-defined indices of similarity $\Delta h$ and $\Delta E^*ab_{ps}$ are used to represent

$$0 \leq \Delta h < \alpha$$

$$0 \leq \Delta E^*ab_{ps} < \beta$$

(wherein $\alpha$ and $\beta$ are positive real numbers) and thereby the similarity of the optical properties of both pearl pigment and spherical silica can be evaluated.

The structural color of the spherical silica with which the pearl pigment is coated is not limited, and the interference color due to reflection of the pearl pigment and the structural color due to reflection of the spherical silica do not have to match, but to further enhance the effect, it is preferable that the interference color due to reflection of the pearl pigment and the structural color due to reflection of the spherical silica are similar. Preferably, the hue angle difference Δh between the interference color due to reflection of the pearl pigment and the structural color due to reflection of the spherical silica is in the range of 0 to 90, and more preferably Δh is in the range of 0 to 45.

For example, when the composite powder of the present invention is blended into foundation or the like, using a composite powder made of a pearl pigment with a red interference color and spherical silica with a red structural color is expected to have the effect of making skin appear brighter and more rosy while suppressing the gloss caused by the interference color of the pearl pigment. In addition to the skin-brightening effect, combining the appropriate pearl pigment with spherical silica is also expected to have a color tone correction effect on skin with color tone problems.

As long as the desired effect of the present invention is achieved, the spherical silica may be electrostatically adsorbed, but it is preferable that the spherical silica is chemically fixed on the pearl pigment with a binder. The binder for fixing the spherical silica on the pearl pigment is not particularly limited, and a resin or silica is preferable. Using a resin or silica as a binder to fix spherical silica on the surface of the pearl pigment to form a composite powder causes the spherical silica to be fixed on the surface of the pearl pigment, making it less likely to fall off from the surface of the pearl pigment, allowing a more favorable in-use feeling to be imparted. The use of a binder prevents the spherical silica fixed on the pearl pigment from peeling off due to mechanical stress during cosmetic manufacturing, allowing the product to remain effective after the cosmetic manufacturing process.

When silica is used as a binder, the silica may be in the form of a film or particles, and may be partially or completely adhered to the surface of the pearl pigment and/or the surface of the spherical silica. Silica may be non-porous or porous. Porous silica may be microporous, mesoporous, or macroporous. Mesoporous silica has fine pores and a large specific surface area, which provides the effect of maintaining the effect of the cosmetic coating film by scattering light and sebum absorbing oil. The silica used as a binder is not particularly limited, and silica obtained by the hydrolysis and condensation reaction of tetraalkoxysilane is preferable.

Examples of the resin that is used as a binder include condensates of trialkoxysilanes such as methyltrimethoxysilane, methyltriethoxysilane, ethyltrimethoxysilane, ethyltriethoxysilane, n-propyltrimethoxysilane, isopropyltrimethoxysilane, isopropyltriethoxysilane, butyltrimethoxysilane, butyltriethoxysilane, hexyltrimethoxysilane, trifluoropropyltrimethoxysilane, trifluoropropyltriethoxysilane, heptadecafluorodecyltrimethoxysilane, and heptadecafluorodecyltriethoxysilane. Preferable examples thereof include condensates of methyltrimethoxysilane and methyltriethoxysilane, and more preferable examples thereof include a condensate of methyltrimethoxysilane.

The amount of binder is not particularly limited, and is preferably in the range of 5 to 50 parts by mass, more preferably 10 to 40 parts by mass, and still more preferably 15 to 30 parts by mass, based on 100 parts by mass of the total amount of the pearl pigment and the spherical silica. An amount of 5 parts by mass or more ensures sufficient fixation of the spherical silica on the surface of the pearl pigment, while an amount of 50 parts by mass or less prevents the product from feeling hard to the touch and preventing aggregation of the powder.

In addition, fixing the spherical silica on the pearl pigment via a binder requires a fixation in the presence of one or more cationic compounds selected from cationic surfactants and cationic water-soluble polymer compounds. This cationic compound not only acts as a catalyst in fixing the spherical silica, but also as a dispersant for the pearl pigment.

The cationic compound is one or more selected from cationic surfactants and cationic water-soluble polymer compounds.

Examples of the cationic surfactants include alkyltrimethylammonium salts, dialkyldimethylammonium salts, polyoxyethylene alkyldimethylammonium salts, dipolyoxyethylene alkylmethylammonium salts, tripolyoxyethylene alkylammonium salts, alkylbenzyldimethylammonium salts, alkylpyridinium salts, monoalkylamine salts, and monoalkylamidoamine salts.

Examples of the cationic water-soluble polymer compounds include dimethyldiallylammonium chloride polymers, vinylimidazoline polymers, methylvinylimidazolium chloride polymers, ethyl acrylate trimethylammonium chloride polymers, ethyl methacrylate trimethylammonium chloride polymers, acrylamidopropyl trimethylammonium chloride polymers, methacrylamidopropyl trimethylammonium chloride polymers, epichlorohydrin/dimethylamine polymers, ethyleneimine polymers, quaternized ethyleneimine polymers, allylamine hydrochloride polymers, and a derivative obtained by copolymerizing polylysine, cationic starch, cationic cellulose, or chitosan with a monomer having a nonionic or anionic group.

The amount of the cationic compound is preferably 0.01 to 2 parts by mass, and more preferably 0.02 to 1 part by mass, based on 100 parts by mass of water used in the fixation reaction. Within this range, spherical silica is sufficiently fixed on the surface of the pearl pigment.

The composite powder of the present invention combines a pearl pigment with spherical silica, thereby allowing impartation of the smoothness and the effect of correcting skin irregularities by the spherical silica on the surface, allowing unnatural luster to be suppressed by the effect of the pearl pigment and the spherical silica, allowing impartation of natural, transparent finish in a case of blending into foundations or the like, and allowing impartation of the effect of brightening the skin. In addition to the effect of brightening the skin, combining the appropriate pearl pigment with spherical silica is expected to have a color tone correction effect on skin with color tone problems. Further, the use of a binder increases the strength of the composite powder, making it possible to withstand the mechanical stresses expected in the cosmetic manufacturing process, thereby maintaining the effects of the composite powder of the present invention.

In order to impart or improve water repellency and improve dispersibility in oil agents, the surface of the composite powder of the present invention is preferably subjected to a hydrophobic surface treatment using a silylating agent, a silicone oil, a wax, paraffin, an organic fluorine compound, a surfactant, and the like. That is, it is preferable that the composite powder is the one subjected to a hydrophobic surface treatment.

The treatment agent used for hydrophobic surface treatment (hydrophobic treatment agent) is not particularly limited as long as it can impart hydrophobicity, and examples thereof include the treatment agents such as silicone treatment agents, waxes, paraffins, organofluorine compounds including perfluoroalkyl and phosphates, surfactants, amino acids including N-acyl glutamic acid, and metal soaps such as aluminum stearate and magnesium myristate.

Silicone treatment agents are more preferable, and examples thereof include: silanes or silylating agents such as triethoxycaprylylsilane (INCI), trimethoxysilyl dimethicone (INCI), trimethylhydroxysilane, chlorosilane, alkoxysilane, disiloxane, and disilazane; dimethicone (INCI), methicone (INCI), hydrogen dimethicone (INCI), triethoxysilylethyl polydimethylsiloxyethyl dimethicone (INCI), triethoxysilylethyl polydimethylsiloxyethylhexyl dimethicone (INCI), and (acrylates/tridecyl acrylate/triethoxysilylpropyl methacrylate/dimethicone methacrylate) copolymer (: labeling name (INCI: Acrylates/Tridecyl Acrylate/Triethoxysilylpropyl Methacrylate/Dimethicone Methacrylate Copolymer)). In particular, treatment with silanes is preferable because of obtaining the effects of suppressing pearly gloss and brightening the skin.

Specific examples of these silicone treatment agents include AES-3083, KF-99P, KF-9901, KF-9908, KF-9909, KP-574, and KP-541 manufactured by Shin-Etsu Chemical Co., Ltd.

Even if the dispersion medium for dispersing the powder is a silicone, a hydrocarbon, or a mixture thereof, from the viewpoint of high affinity, triethoxysilylethyl polydimethylsiloxyethyl hexyl dimethicone (KF-9909, manufactured by Shin-Etsu Chemical Co., Ltd.), which is a dimethylpolysiloxane having a triethoxysilyl group, a polydimethylsiloxyethyl group, and a hexyl group on its side chain, and the like are also preferable.

Further, the above hydrophobic treatment agents can be used singly or in appropriate combination of two or more.

[Surface treatment method]

The method for surface treating the composite powder of the present invention with a hydrophobic treatment agent is not particularly limited, and any known method can be used. Surface treatment methods can be broadly divided into dry methods and wet methods. In the dry method, the treatment can be performed by mixing/contacting the composite powder of the present invention with the hydrophobic treatment agent using any stirrer, grinder, mixer, disperser, or the like, such as a Henschel mixer, a ball mill, a jet mill, a kneader, a planetary mixer, a sand mill, an attritor, a ribbon blender, a disper mixer, or a homomixer. In this case, the treatment may be performed while applying energy such as heat, mechanochemical mechanical force, or superheated steam. In addition, after the composite powder of the present invention and the hydrophobic treatment agent are thoroughly mixed/contacted, energy such as heat, mechanochemical mechanical force, or superheated steam may be applied separately to the powder for treatment. In addition, when the hydrophobic treatment agent is mixed/contacted with the composite powder of the present invention, in order to improve the dispersion efficiency of the hydrophobic treatment agent, a means may be used, for example, the hydrophobic treatment agent is dissolved or dispersed previously in an arbitrary amount of water, solvent, or supercritical fluid, and then this mixture is sprayed onto the composite powder of the present invention. In the wet method, the composite powder of the present invention and a hydrophobic treatment agent are dispersed in water, a solvent, or a supercritical fluid, and are mixed/contacted, and then the solvent is evaporated, and energy such as heat, mechanochemical mechanical force, or superheated steam can be further separately applied to perform the treatment.

To effectively use the composite powder of the present invention, it is preferable to arrange the composite powder of the present invention in a manner close to a monolayer on the surface of the skin or the surface of a cosmetic coating film. For example, when the composite powder of the present invention is used in a foundation or the like, it is preferable to spread the composite powder thinly and evenly. In addition, when the composite powder of the present invention is used in a finishing cosmetic or touch-up cosmetic, the effects of the present invention are more effectively achieved by an application method that forms a thin film of the composite powder.

Herein, the effect of improving color tone problems by the composite powder of the present invention will be described in detail. The composite powder of the present invention can interfere with light of any wavelength and produce color through the optical interference effect caused by the structure thereof. That is, when white light is incident on the composite powder of the present invention, the reflected interference light is different, and is converted into light of wavelengths near the complementary color of the scattered light from areas recognized as skin color tone problems, thereby allowing those skin color tone problems to be less noticeable.

To achieve the effect of concealing yellow-red color tone problems among color tone problems, it is preferable to blend the composite powder of the present invention into cosmetics so as to make a configuration having a maximum reflection peak at wavelengths of 400 to 550 nm (blue-green). In addition, to achieve the effect of concealing blue-green color tone problems, it is preferable to blend the composite powder of the present invention into cosmetics so as to make a configuration having a maximum reflection peak at wavelengths of 550 to 750 nm (yellow-red).

For example, to eliminate color tone problems that are recognized as yellow-red color tone problems over a wide range of skin (dullness, red face, and the like), that is, color tone problems in which scattered light has a wavelength range of 580 to 700 nm (yellow-red) and a wide range of broad reflection peaks, it is preferable to use a composite powder of the present invention that can have a reflection peak in the wavelength of 475 to 550 nm (blue-green).

In addition, color tone problems can be more effectively eliminated by setting the color tone reflected by the composite powder of the present invention such that the reflected light selected and employed for color tone problems shifts to the longer wavelength side when eliminating "dullness" and the reflected light selected and employed for color tone problems shifts to the shorter wavelength side when eliminating "red face".

[Spherical powder]

The spherical powder used in the composite powder of the present invention will be described. Non-spherical particles used in cosmetics does not act as a type of ball bearing during application of cosmetics, failing to achieve the effect of dramatically improving the smoothness of the composite powder. In addition, it is difficult to achieve the effect of effectively scattering light on the surface of the cosmetic coating film and concealing skin morphological problems related to light reflection (soft focus effect).

The term "spherical" in the spherical powder of the present invention is as explained above in relation to spherical silica.

Any known spherical powder can be used, and is not particularly limited, and spherical silica is preferable because powder with a high degree of monodispersity can be manufactured.

[Method for manufacturing composite powder]

The composite powder of the present invention can be manufactured, for example, by stirring a pearl pigment and hydrophilic spherical silica in a mixer and then fixing them together using electrostatic force. Note that the type of mixer used is not particularly limited, and examples thereof include a Sample Mill (Kyoritsu Riko Co., Ltd.).

In addition, for example, the composite powder of the present invention can be manufactured by uniformly mixing the pearl pigment with ion-exchanged water, then adding a dispersion with spherical silica dispersed in the presence of one or more cationic compounds selected from cationic surfactants and cationic water-soluble polymer compounds, adding aqueous ammonia to make the system alkaline, adding a binder, and then filtering the aged mixture. This method, for example, provides a composite powder in which the spherical silica is chemically fixed on the pearl pigment with the binder.

[Cosmetic]

The present invention is a cosmetic including the composite powder described above. The cosmetic of the present invention is used in a variety of cosmetics, and is particularly suitable for cosmetics applied to the skin such as skin care cosmetics, makeup cosmetics, antiperspirant cosmetics, and UV protection cosmetics; cosmetics applied to the hair such as hair cosmetics; and nail cosmetics.

Examples of skin care cosmetics include lotions, emulsions, creams, cleansers, packs, oil liquids, massage products, beauty serums, beauty oils, detergents, deodorants, hand creams, lip balms, and wrinkle concealers.

Examples of the makeup cosmetics include makeup primers, foundations, concealers, face powders, cheek colors, eye colors, eye shadows, mascaras, eyeliners, eyebrow pencils, and lipsticks.

Examples of the antiperspirant cosmetics include roll-on type, cream type, solution type, and stick type antiperspirant cosmetics.

Examples of UV protection cosmetics include sunscreen oil, sunscreen lotion, and sunscreen cream.

Examples of hair cosmetics include shampoos, rinses, treatments, and setting agents.

The cosmetics of the present invention may be in any form of, for example, a powder, oily liquid, water-in-oil emulsion, oil-in-water emulsion, non-aqueous emulsion, or multiple emulsions such as W/O/W or O/W/O. In addition, the properties of the cosmetics of the present invention can be selected from a variety of properties such as liquid, emulsion, cream, solid, paste, gel, powder, pressed, multi-layered, mousse, spray, stick, and pencil.

The cosmetics of the present invention can contain various ingredients used in common cosmetics, depending on the intended purpose. For example, the cosmetics of the present invention may include (1) an oil agent, (2) an aqueous ingredient, (3) a surfactant, (4) a powder other than that of the present invention, (5) a composition including a crosslinked organopolysiloxane and an oil agent that is liquid at room temperature, (6) a film-forming agent, (7) an ultraviolet absorbing and scattering agent, and (8) other additives. These ingredients may be used singly or in appropriate combinations of two or more.

(1) Oil agent

The oil agent may be volatile or non-volatile, and may be solid, semi-solid, or liquid at room temperature (25°C), and examples thereof include silicone oils, waxes, natural animal and vegetable oils and fats and semi-synthetic oils and fats, hydrocarbon oils, higher alcohols, higher fatty acids, ester oils, fluorine-based oil agents, and ultraviolet absorbers.

· Silicone oil

Examples of the silicone oils include: dimethicone (INCI), trisiloxane (INCI), alkyl-modified silicones, such as methyl trimethicone (INCI), ethyl trisiloxane (INCI), ethyl methicone (INCI), and hexyl dimethicone (INCI); long-chain alkyl-modified silicones such as caprylyl methicone (INCI); low to high viscosity linear or branched organopolysiloxanes such as phenyl trimethicone (INCI), diphenyl dimethicone (INCI), diphenylsiloxyphenyl trimethicone (INCI), tetraphenyldimethyldisiloxane (INCI), and methylhydrogenpolysiloxane; cyclic organopolysiloxanes such as cyclotetrasiloxane (INCI), cyclopentasiloxane (INCI), and cyclohexasiloxane (INCI); amino-modified organopolysiloxanes such as amodimethicone (INCI) and aminopropyl dimethicone (INCI); pyrrolidone-modified organopolysiloxanes such as PCA dimethicone (INCI); pyrrolidone carboxylic acid-modified organopolysiloxane, high-polymerization gum-like dimethylpolysiloxane, gum-like amino-modified organopolysiloxane; silicone rubber such as gum-like dimethylsiloxane-methylphenylsiloxane copolymer; low viscosity organopolysiloxane solutions of silicone gums and rubbers; amino acid-modified silicone, fluorine-modified silicone, silicone resin, and dissolved silicone resin.

Examples of commercially available silicone oils include KF-96L-1cs, KF-96L-1.5cs, KF-96L-2cs, KF-96A-6cs, KF-4422, KF-4418, KF-54, KF-54HV, KF-56A, and KF-995 manufactured by Shin-Etsu Chemical Co., Ltd.

· Solid oily ingredients

In the present invention, when it is desired to solidify the cosmetic, it is preferable to blend an oily ingredient that is solid at 25°C. Oily ingredients that are solid at 25°C preferably have a melting point of 40°C or more, more preferably 60 to 110°C, and examples thereof include waxes, hydrocarbon oils, esters, higher alcohols, and higher fatty acids, and these ingredients are not particularly limited as long as they can typically be blended in cosmetics. Specific examples thereof include vegetable waxes such as carnauba wax (INCI: Copernicia Cerifera (Carnauba) Wax), sugarcane wax, candelilla wax (INCI: Euphorbia Cerifera (Candelilla) Wax), refined candelilla wax, rice wax, wood wax, Jojoba wax, kapok wax, rice bran wax, white bayberry fruit wax, shea butter, cocoa butter, Japan wax (INCI: Rhus Succedanea Fruit Wax), montan wax (INCI: Montan Wax), and hydrogenated castor oil isostearate; animal waxes such as beeswax, beef tallow, beef bone fat, pork fat (INCI: Lard), horse fat (INCI: Horse Fat), sheep fat, lanolin (INCI: Lanolin), insect wax, shellac wax, and spermaceti; semi-synthetic waxes such as lanolin esters, lanolin fatty acid esters, and beeswax esters; hydrogenated oils such as hydrogenated castor oil and hydrogenated coconut oil, hydrocarbon waxes such as solid paraffin, polyethylene, ceresin, ozokerite, and microcrystalline wax; wax esters such as synthetic beeswax; amino acid stearyl alcohols such as dioctyldodecyl lauroyl glutamate; higher fatty acids such as stearic acid and behenic acid; and silicone waxes such as acrylic-silicone graft or block copolymer acrylic silicone resins (acrylic-silicone graft copolymers: KP-561P, 562P, and the like, manufactured by Shin-Etsu Chemical Co., Ltd.), or derivatives thereof, and preferably, one or more selected from these are used.

· Natural animal and vegetable oils and fats and semi-synthetic oil agents

Examples of the natural animal and vegetable oils and fats and semi-synthetic oil agents include natural vegetable oils, such as avocado oil (: labeling name (INCI: Persea Gratissima (Avocado) Oil)), linseed oil (: labeling name (INCI: Linum Usitatissimum (Linseed) Seed Oil)), almond oil (: labeling name (INCI: Prunus Amygdalus Dulcis (Sweet Almond) Oil)), perilla oil (labeling name), olive oil (: labeling name (INCI: Olea Europaea (Olive) Fruit Oil)), Torreya californica oil (: labeling name (INCI: Torreya Californica (California Nutmeg) Oil)), citronella oil (: labeling name (INCI: Cymbopogon Nardus (Citronella) Oil)), Torreya nucifera seed oil (: labeling name (INCI: Torreya Nucifera Seed Oil)), apricot kernel oil (: labeling name (INCI: Kyounin Yu)), Wheat germ oil (: labeling name (INCI: Triticum Vulgare (Wheat) Germ Oil)), germ oils such as rice germ oil (: labeling name (INCI: Oryza Sativa (Rice) Germ Oil)) and corn germ oil (: labeling name (INCI: Zea Mays (Corn) Germ Oil)), sesame oil (: labeling name (INCI: Sesamum Indicum (Sesame) Seed Oil)), Rice bran oil (: labeling name (INCI: Oryza Sativa (Rice) Bran Oil)), sasanqua oil (: labeling name (INCI: Camellia Kissi Seed Oil)), safflower oil (: labeling name (INCI: Carthamus Tinctorius (Safflower) Seed Oil)), soybean oil (: labeling name (INCI: Glycine Soja (Soybean) Oil)), tea seed oil (: labeling name (INCI: Camellia Sinensis Seed Oil)), camellia oil (: labeling name (INCI: Camellia Japonica Seed Oil)), evening primrose oil (: labeling name (INCI: Oenothera Biennis (Evening Primrose) Oil)), rapeseed oil (labeling name), persic oil (labeling name), palm oil (: labeling name (INCI: Elaeis Guineensis (Palm) Oil)), palm kernel oil (: labeling name (INCI: Elaeis Guineensis (Palm) Kernel Oil)), castor oil (: labeling name (INCI: Ricinus Communis (Castor) Seed Oil)), sunflower seed oil (: labeling name (INCI: Helianthus Annuus (Sunflower) Seed Oil)), grape seed oil (: labeling name (INCI: Vitis Vinifera (Grape) Seed Oil)), jojoba seed oil (: labeling name (INCI: Simmondsia Chinensis (Jojoba) Seed Oil)), Macadamia Seed Oil (: labeling name (INCI: Macadamia Ternifolia Seed Oil)), meadowfoam oil (: labeling name (INCI: Limnanthes Alba (Meadowfoam) Seed Oil)), cottonseed oil (: labeling name (INCI: Gossypium Herbaceum (Cotton) Seed Oil)), coconut oil (: labeling name (INCI: Cocos Nucifera (Coconut) Oil)), and peanut oil (: labeling name (INCI: Arachis Hypogaea (Peanut) Oil)); natural animal oils such as shark liver oil (: labeling name (INCI: Shark Liver Oil)), cod liver oil (: labeling name (INCI: Cod Liver Oil)), fish liver oil (: labeling name (INCI: Fish Liver Oil)), turtle oil (: labeling name (INCI: Turtle Oil)), mink oil (: labeling name (INCI: Mink Oil)), and egg yolk oil (: labeling name (INCI: Egg Oil)); and semi-synthetic oils such as hydrogenated coconut oil (:labeling name (INCI: Hydrogenated Coconut Oil)), and liquid lanolin (: labeling name (INCI: Lanolin Oil)).

· Hydrocarbon oil

Examples of hydrocarbon oils include linear and branched hydrocarbon oils, and may be either volatile hydrocarbon oils or non-volatile hydrocarbon oils. Specific examples thereof include alkanes such as olefin oligomers (INCI), isoparaffins such as (C13, 14) isoparaffin (INCI), isododecane (INCI), undecane (INCI), dodecane (INCI), isohexadecane (INCI), hydrogenated polyisobutene (: labeling name (INCI: Hydrogenated Polyisobutene)), squalane (INCI), mineral oil (INCI), palm alkane (INCI), and (C13 to 15) alkanes (INCI).

· Higher alcohol

Examples of higher alcohols include straight-chain saturated alcohols having 6 or more carbon atoms, such as lauryl alcohol (INCI), hexyldecanol (INCI), oleyl alcohol (INCI), isostearyl alcohol (INCI), octyldodecanol (INCI), decyltetradecanol (INCI), myristyl alcohol (INCI), cetyl alcohol (INCI), stearyl alcohol (INCI), and behenyl alcohol (INCI); and batyl alcohol (INCI). In addition, examples thereof include sterols, such as cholesterol (INCI), sitosterol (: labeling name (INCI: Beta-Sitosterol)), phytosterols (INCI), and lanosterol (INCI).

· Ester oil

Examples of the ester oils include diisobutyl adipate (: labeling name (INCI: Diisobutyl Adipate)), dihexyldecyl adipate (labeling name), diheptylundecyl adipate (: labeling name (INCI: Diheptylundecyl Adipate)); alkyl glycol monoisostearates such as isostearyl isostearate (: labeling name (INCI: Isostearyl Isostearate)); hexyldecyl isostearate (: labeling name (INCI: Hexyldecyl Isostearate)), trimethylolpropane triisostearate (: labeling name (INCI: Trimethylolpropane Triisostearate)), glycol diethylhexanoate (: labeling name (INCI: Glycol Diethylhexanoate)), cetyl ethylhexanoate (: labeling name (INCI: Cetyl Ethylhexanoate)), trimethylolpropane triethylhexanoate (: labeling name (INCI: Trimethylolpropane Triethylhexanoate)), pentaerythrityl tetraethylhexanoate (: labeling name (INCI: Pentaerythrityl Tetraethylhexanoate)); octyldodecyl esters such as octyldodecyl stearoyloxystearate (: labeling name (INCI: Octyldodecyl Stearoyl Stearate)); oleyl oleate (: labeling name (INCI: Oleyl Oleate)), octyldodecyl oleate (: labeling name (INCI: Octyldodecyl Oleate)), decyl oleate (: labeling name (INCI: Decyl Oleate)), neopentyl glycol dioctanoate (: labeling name (INCI: Neopentyl Glycol Diethylhexanoate)), neopentyl glycol dicaprate (: labeling name (INCI: Neopentyl Glycol Dicaprate)), diisostearyl malate (: labeling name (INCI: Diisostearyl Malate)), triethyl citrate (: labeling name (INCI: Triethyl Citrate)), diethylhexyl succinate (: labeling name (INCI: Diethylhexyl Succinate)), amyl acetate (: labeling name (INCI: Amyl Acetate)), ethyl acetate (: labeling name (INCI: Ethyl Acetate)), butyl acetate (: labeling name (INCI: Butyl Acetate)), isocetyl stearate (: labeling name (INCI: Isocetyl Stearate)), butyl stearate (: labeling name (INCI: Butyl Stearate)), diisopropyl sebacate (: labeling name (INCI: Diisopropyl Sebacate)), diethylhexyl sebacate (: labeling name (INCI: Diethylhexyl Sebacate)), Cetyl lactate (: labeling name (INCI: Cetyl Lactate)), myristyl lactate (: labeling name (INCI: Myristyl Lactate)), isononyl isononanoate (: labeling name (INCI: Isononyl Isononanoate)), isotridecyl isononanoate (: labeling name (INCI: Isotridecyl Isononanoate)); palmitate esters, such as isopropyl palmitate (: labeling name (INCI: Isopropyl Palmitate)), ethylhexyl palmitate (: labeling name (INCI: Ethylhexyl Isopalmitate)), and hexyldecyl palmitate (: labeling name (INCI: Isocetyl Palmitate, Hexyldecyl Palmitate)); cholesteryl hydroxystearate (: labeling name (INCI: Cholesteryl Hydroxystearate)); myristate esters, such as isopropyl myristate (: labeling name (INCI: Isopropyl Myristate)), octyldodecyl myristate (: labeling name (INCI: Octyldodecyl Myristate)), and myristyl myristate (: labeling name (INCI: Myristyl Myristate)); and ethylhexyl laurate (: labeling name (INCI: Ethylhexyl Laurate)), hexyl laurate (: labeling name (INCI: Hexyl Laurate)), dioctyldodecyl lauroyl glutamate (: labeling name (INCI: Dioctyldodecyl Lauroyl Glutamate)), isopropyl lauroyl sarcosinate (: labeling name (INCI: Isopropyl Lauroyl Sarcosinate)), and Coco-Caprylate/Caprate (: labeling name (INCI: Coco-Caprylate/Caprate)).

In addition, among ester oils, the glyceride oils include triethylhexanoin (INCI), caprylic/capric triglyceride (: labeling name (INCI: Caprylic/Capric Triglyceride)), cocoglycerides (INCI), caprylic/capric/succinic triglyceride (: labeling name (INCI: Caprylic/Capric/Succinic Triglyceride)), and caprylic/capric glycerides (: labeling name (INCI: Caprylic/Capric Glycerides)).

· Fluorine-based oil agent

Examples of the fluorine-based oil agents include perfluorodecalin (INCI), perfluorononyl dimethicone (INCI), and perfluoromethylcyclopentane (INCI).

· Ultraviolet absorber

Examples of the ultraviolet absorbers include oxybenzone-1 (: labeling name (INCI: Benzophenone-1)), oxybenzone-2 (: labeling name (INCI: Benzophenone-2)), oxybenzone-3 (: labeling name (INCI: Benzophenone-3)), oxybenzone-4 (: labeling name (INCI: Benzophenone-4)), oxybenzone-5 (: labeling name (INCI: Benzophenone-5)), oxybenzone-6 (: labeling name (INCI: Benzophenone-6)), oxybenzone-9 (: labeling name (INCI: Benzophenone-9)), homosalate (INCI), octocrylene (INCI), t-butyl methoxydibenzoylmethane (: labeling name (INCI: Butyl Methoxydibenzoylmethane)), ethylhexyl salicylate (: labeling name (INCI: Ethylhexyl Salicylate)), diethylamino hydroxybenzoyl hexyl benzoate (: labeling name (INCI: Diethylamino Hydroxybenzoyl Hexyl Benzoate)), polysilicone-15 (INCI), ethylhexyl dimethoxybenzylidene dioxoimidazolidine propionate (: labeling name (INCI: Ethylhexyl Dimethoxybenzylidene Dioxoimidazolidine Propionate)), terephthalylidene dicamphor sulfonic acid (: labeling name (INCI: Terephthalylidene Dicamphor Sulfonic Acid)), ethylhexyl triazone (INCI), methyl bis(trimethylsiloxy)silyl isopentyl trimethoxycinnamate (: labeling name (INCI: Isopentyl Trimethoxycinnamate Trisiloxane)), drometrizole trisiloxane (INCI), ethylhexyl dimethyl PABA (: labeling name (INCI: Ethylhexyl Dimethyl PABA)), isopropyl para-methoxycinnamate (: labeling name (INCI: Isopropyl Methoxycinnamate)), ethylhexyl methoxycinnamate (: labeling name (INCI: Ethylhexyl Methoxycinnamate)), bis-ethylhexyloxyphenol methoxyphenyl triazine (INCI), phenylbenzimidazole sulfonic acid (: labeling name (INCI: Phenylbenzimidazole Sulfonic Acid)), Methylene bis-benzotriazolyl tetramethylbutylphenol (INCI), glyceryl ethylhexanoate dimethoxycinnamate (: labeling name (INCI: Glyceryl Ethylhexanoate Dimethoxycinnamate)), glyceryl PABA (INCI), diisopropyl methyl cinnamate (: labeling name (INCI: Diisopropyl Methyl Cinnamate)), and cinoxate (INCI). In addition, it is possible to use a UVA absorber (for example, diethylaminohydroxybenzoyl hexyl benzoate, and the like) and a UVB absorber (for example, ethylhexyl methoxycinnamate, and the like) in combination, and these can also be combined in any desired manner.

(2) Aqueous ingredient

The aqueous ingredient is not particularly limited as long as it is an aqueous ingredient that can typically be blended in cosmetics. Specific examples thereof include water, lower alcohols, preferably having 2 to 5 carbon atoms, such as ethanol (: labeling name (INCI: Alcohol)) and isopropanol (: labeling name (INCI: Isopropyl Alcohol)), and sugar alcohols, such as sorbitol (INCI), maltose (INCI), and xylitol (INCI). In addition, examples thereof include: polyhydric alcohols such as BG (: labeling name (INCI: Butylene Glycol)), PG (: labeling name (INCI: Propylene Glycol)), DPG (: labeling name (INCI: Dipropylene Glycol)), pentylene glycol (INCI), 1,10-decanediol (INCI), octanediol (INCI), 1,2-hexanediol (INCI), erythritol (INCI), glycerin (INCI), diglycerin (INCI), and polyethylene glycol; and moisturizing agents such as glucose (INCI), glyceryl glucoside (INCI), betaine (INCI), sodium chondroitin sulfate (: labeling name (INCI: Sodium chondroitin sulfate)), PCA Na (: labeling name (INCI: Sodium PCA)), methyl gluceth-10 (INCI), methyl gluceth-20 (INCI), hyaluronic acid, egg yolk lecithin, soy lecithin, phosphatidylcholine, phosphatidylethanolamine, phosphatidylserine, phosphatidylglycerol, phosphatidylinositol, and sphingolipids. In addition, examples thereof include: water-soluble polymer compounds such as gum arabic, guar gum, carrageenan, agar, quince seed, locust bean gum, xanthan gum, pullulan, sodium carboxymethylcellulose, hydroxyethyl cellulose, vinyl polymers such as carboxyvinyl polymers; and acrylic polymers such as (ammonium acryloyldimethyltaurate/vinylpyrrolidone) copolymer, (sodium acrylate/sodium acryloyldimethyltaurate) copolymer, (hydroxyethyl acrylate/sodium acryloyldimethyltaurate) copolymer, (acrylamide/sodium acryloyldimethyltaurate) copolymer, and polyacrylamide.

(3) Surfactant

The surfactants include nonionic, anionic, cationic, and amphoteric surfactants, but there are no particular limitations and any surfactants used in common cosmetics can be used. Among these surfactants, one or more selected from non-crosslinked silicone surfactants or crosslinked silicone surfactants are preferable, because they allow for the stable cosmetics. In either case, the amount of surfactant blended is preferably 0.1 to 20% by mass of the total cosmetic. Amounts of 0.1% by mass or more ensure sufficient dispersion and emulsification functions, while amounts of 20% by mass or less are preferable because they prevent the cosmetic from having a sticky in-use feeling. The HLB of the surfactant is not limited, and is preferably 2 to 14.5 in order to maintain the water resistance of the cosmetic.

The non-crosslinked silicone surfactants are those in which a part of the methyl groups in the linear or branched silicone main chain are substituted with hydrophilic groups such as polyethylene glycol or polyglycerin, and specifically it is preferably linear or branched polyoxyethylene-modified organopolysiloxane, linear or branched polyoxyethylene-polyoxypropylene-modified organopolysiloxane, linear or branched polyoxyethylene-alkyl co-modified organopolysiloxane, linear or branched polyoxyethylene-polyoxypropylene/alkyl co-modified organopolysiloxane, linear or branched polyglycerin-modified organopolysiloxane, linear or branched polyglycerin/alkyl co-modified organopolysiloxane, or linear or branched pyrrolidone-modified organopolysiloxane. Examples thereof include PEG-11 methyl ether dimethicone (INCI), PEG/PPG-20/22 butyl ether dimethicone (INCI), PEG-3 dimethicone (INCI), PEG-10 dimethicone (INCI), PEG-9 polydimethylsiloxyethyl dimethicone (INCI), lauryl PEG-9 polydimethylsiloxyethyl dimethicone (INCI), cetyl PEG/PPG-10/1 dimethicone (INCI), polyglyceryl-3 disiloxane dimethicone (INCI), polyglyceryl-3 polydimethylsiloxyethyl dimethicone (INCI), lauryl polyglyceryl-3 polydimethylsiloxyethyl dimethicone (INCI), and bis-butyl dimethicone polyglyceryl-3 (INCI).

Examples of the commercially available products include KF-6011, KF-6011P, KF-6012, KF-6015, KF-6017, KF-6043, KF-6028, KF-6038, KF-6048, KF-6100, KF-6104, KF-6106, KF-6105, and KF-6115 manufactured by Shin-Etsu Chemical Co., Ltd.

Examples of the cross-linked silicone surfactants include: cross-linked polyether-modified silicones such as dimethicone/(PEG-10/15) crosspolymer (INCI), PEG-15/lauryl dimethicone crosspolymer (INCI), PEG-10/lauryl dimethicone crosspolymer (INCI), and PEG-15/lauryl polydimethylsiloxyethyl dimethicone crosspolymer (INCI); and crosslinked polyglycerin-modified silicones, such as dimethicone/polyglycerin-3 crosspolymer (INCI), lauryl dimethicone/polyglycerin-3 crosspolymer (INCI), and polyglycerin-3/lauryl polydimethylsiloxyethyl dimethicone crosspolymer (INCI).

In addition, when the crosslinked silicone surfactant is used, in a composition including the crosslinked silicone surfactant and an oil agent that is liquid at room temperature, it is preferable that the crosslinked silicone surfactant swells by including the liquid oil agent in an amount equal to or more than its own weight based on the liquid oil agent.

As the above-described liquid oil agent, among the optional ingredients of (1) oil agent, liquid silicone oils, hydrocarbon oils, ester oils, natural animal and vegetable oils and fats, semi-synthetic oils and fats, fluorine-based oil agents, and the like can be used, and examples thereof include cyclopentasiloxane (INCI), dimethicone (INCI), mineral oil (INCI), isododecane (INCI), isohexadecane (INCI), triethylhexanoin (INCI), isotridecyl isononanoate (: labeling name (INCI: Isotridecyl Isononanoate)), and squalane (INCI).

Examples of the commercially available cross-linked silicone surfactants that swell by including liquid oil agents include KSG-210, KSG-240, KSG-270, KSG-310, KSG-320, KSG-330, KSG-340, KSG-320Z, KSG-350Z, KSG-710, KSG-810, KSG-820, KSG-830, KSG-840, KSG-820Z, and KSG-850Z, manufactured by Shin-Etsu Chemical Co., Ltd.

(4) Powders other than those of the present invention

Powders other than the composite powder of the present invention can be blended. Examples of the powders include colored pigments, inorganic powders, inorganic colored pearl pigments, metal powders, organic powders, metal soaps, organic dyes, and inorganic-organic composite powders. Specific examples thereof are as follows.

· Colored pigment

The colored pigment is not particularly limited, as long as it is a pigment that is typically used for the purpose of coloring cosmetics, and any of colored pigments such as red iron oxide (: labeling name (INCI: Iron Oxides)), yellow iron oxide (: labeling name (INCI: Iron Oxides)), white titanium dioxide (: labeling name (INCI: Titanium Dioxide)), black iron oxide (: labeling name (INCI: Iron Oxides)), ultramarines (: labeling name (INCI: Ultramarines)), ferric ferrocyanide (: labeling name (INCI: Ferric Ferrocyanide, Ferric Ammonium Ferrocyanide)), manganese violet (: labeling name (INCI: Manganese Violet)), cobalt titanate (: labeling name (INCI: Cobalt Titanium Oxide)), chromium hydroxide (: labeling name (INCI: Chromium Hydroxide Green)), chromium oxide (: labeling name (INCI: Chromium Oxide Greens)), aluminum/cobalt oxide (: labeling name (INCI: Cobalt Aluminum Oxide)), cobalt titanate (: labeling name (INCI: Cobalt Titanium Oxide)), titanium/titanium dioxide fired product (: labeling name (INCI: Titanium/Titanium Dioxide)), (Li/cobalt) titanate (: labeling name (INCI: Lithium Cobalt Titanate)), cobalt titanate (: labeling name (INCI: Cobalt Titanium Oxide)), iron oxide/titanium dioxide sintered product (labeling name), composites doped with different metals, such as iron oxide-doped titanium dioxide (: labeling name (INCI: Iron Oxides, Titanium Dioxide)), titanium nitride (: labeling name (INCI: Titanium Nitride)), ferrous hydroxide (: labeling name (INCI: Iron Hydroxide)), inorganic brown pigments such as γ-iron oxide, inorganic yellow pigments such as ochre, lake-coated tar-based pigments, and lake-coated natural pigments can be used.

In addition, the shape of the colored pigment may be any shape, such as spherical, approximately spherical, rod-shaped, spindle-shaped, petal-shaped, strip-shaped, or irregular, and there is no particular limitation on the geometric form as long as it can impart color to the cosmetic.

· Inorganic powder

Examples of the inorganic powder include fine particles composed of zirconium oxide (: labeling name (INCI: Zirconium Dioxide)), zinc oxide (: labeling name (INCI: Zinc Oxide)), cerium oxide (: labeling name (INCI: Cerium Oxide)), magnesium oxide (: labeling name (INCI: Magnesium Oxide)), barium sulfate (: labeling name (INCI: Barium Sulfate)), calcium sulfate (: labeling name (INCI: Calcium Carbonate)), magnesium sulfate (: labeling name (INCI: Magnesium Sulfate)), calcium carbonate (: labeling name (INCI: Calcium Carbonate)), magnesium carbonate (: labeling name (INCI: Magnesium Carbonate)), talc (INCI), mica (INCI), kaolin (INCI), synthetic fluorphlogopite (: labeling name (INCI: Synthetic Fluorphlogopite)), synthetic iron phlogopite (labeling name), biotite (: labeling name (INCI: Biotite)), potassium silicate (: labeling name (INCI: Potassium Silicate)), Silica (INCI), aluminum silicate (: labeling name (INCI: Aluminum Silicate)), magnesium silicate (: labeling name (INCI: Magnesium Silicate)), Al/Mg silicate (: labeling name (INCI: Magnesium Aluminum Silicate)), calcium silicate (: labeling name (INCI: Calcium silicate)), Al/Ca/Na silicate (: labeling name (INCI: Aluminum Calcium Sodium Silicate)), Li/Mg/Na silicate (: labeling name (INCI: Lithium Magnesium Sodium Silicate)), Na/Mg silicate (: labeling name (INCI: Sodium Magnesium Silicate)), Ca/Al borosilicate (: labeling name (INCI: Calcium Aluminum Borosilicate)), Ca/Na borosilicate (: labeling name (INCI: Calcium Sodium Borosilicate)), Hydroxyapatite (INCI), bentonite (INCI), montmorillonite (INCI), hectorite (INCI), zeolite (INCI), alumina (INCI), aluminum hydroxide (: labeling name (INCI: Aluminum Hydroxide)), boron nitride (: labeling name (INCI: Boron Nitride)), or glass (: labeling name (INCI: Glass)).

· Inorganic colored pearl pigment

In addition, examples of the inorganic colored pearl pigments include: pearling agents such as mica (INCI) coated with titanium dioxide (: labeling name (INCI: Titanium Dioxide)), and synthetic fluorphlogopite (: labeling name (INCI: Synthetic Fluorphlogopite)) coated with titanium dioxide (: labeling name (INCI: Titanium Dioxide)); and pearl pigments such as bismuth oxychloride (: labeling name (INCI: Bismuth Oxychloride)), bismuth oxychloride (: labeling name (INCI: Bismuth Oxychloride)) coated with titanium dioxide (: labeling name (INCI: Titanium Dioxide)), talc (INCI) coated with titanium dioxide (: labeling name (INCI: Titanium Dioxide)), fish scale foil (labeling name), and colored mica coated with titanium dioxide (: labeling name (INCI: Titanium Dioxide)), and there are no particular limitations on whether the surface is untreated or has undergone a known surface treatment commonly used in cosmetics.

· Metal powder

Examples of the metal powders include fine metal particles composed of aluminum (: labeling name (INCI: Aluminum, Aluminum Powder)), copper (: labeling name (INCI: Copper Powder)), silver (: labeling name (INCI: Silver Powder)), and gold (: labeling name (INCI: Gold)).

· Organic powder

Examples of the organic powders include powder composed of silicone, polyamide, polyacrylic acid/acrylic ester, polyester, polyethylene (INCI), polypropylene (INCI), polystyrene (INCI), styrene/acrylic acid copolymer, divinylbenzene/styrene copolymer, polyurethane, vinyl resin, urea resin, melamine resin, benzoguanamine, polymethylbenzoguanamine, tetrafluoroethylene, polymethyl methacrylate, cellulose (INCI), silk (INCI), nylon (labeling name), phenol resin, epoxy resin, or polycarbonate.

In particular, examples of the silicones include: silicone resin particles; polymethylsilsesquioxane (INCI), silicone rubber powder, silicone resin-coated silicone rubber powder; vinyl dimethicone/methicone silsesquioxane crosspolymer (INCI), diphenyl dimethicone/vinyl diphenyl dimethicone/silsesquioxane crosspolymer (INCI), polysilicone-1 crosspolymer (INCI), and polysilicone-22 (INCI).

Examples of commercially available powders composed of silicone include KMP-590, KMP-591, KMP-592, KMP-597, KMP-598, KSP-100, KSP-101, KSP-102, KSP-105, KSP-300, KSP-411, KSP-441, KM-9729, and KM-440 manufactured by Shin-Etsu Chemical Co., Ltd.

· Metal soap

In addition, examples include metal soaps, and specific examples of thereof include powder composed of zinc stearate (: labeling name (INCI: Zinc Stearate)), aluminum stearate (: labeling name (INCI: Aluminum Stearate)), calcium stearate (: labeling name (INCI: Calcium Stearate)), magnesium stearate (: labeling name (INCI: Magnesium Stearate)), zinc myristate (: labeling name (INCI: Zinc Myristate)), magnesium myristate (: labeling name (INCI: Magnesium Myristate)), zinc/sodium cetyl phosphate (: labeling name (INCI: Sodium Zinc Cetyl Phosphate)), or potassium cetyl phosphate (: labeling name (INCI: Potassium Cetyl Phosphate)).

· Organic dye

Further examples include the organic dyes, and specific examples thereof include tar dyes such as Red 3, Red 104 (1) (: labeling name (INCI: Red 28, Red 28 Lake)), Red 106, Red 201 (: labeling name (INCI: Red 6)), Red 202 (: labeling name (INCI: Red 7)), Red 204, Red 205, Red 220 (: labeling name (INCI: Red 34)), Red 226 (: labeling name (INCI: Red 30)), Red 227 (: labeling name (INCI: Red 33, RED 33 Lake)), Red 228 (: labeling name (INCI: Red 36)), Red 230 (1) (: labeling name (INCI: Red 22, Red 22 Lake)), Red 230 (2) (labeling name), Red 401 (labeling name), Red 505 (labeling name), Yellow 4 (: labeling name (INCI: Yellow 5)), Yellow 5 (: labeling name (INCI: Yellow 6, Yellow 6 Lake)), Yellow 202 (1) (: labeling name (INCI: Yellow 8)), Yellow 203 (: labeling name (INCI: Yellow 10, Yellow 10 Lake)), Yellow 204 (: labeling name (INCI: Yellow 11)), Yellow 401, Blue 1 (: labeling name (INCI: Blue 1, Blue 1 Lake)), Blue 2, Blue 201, Blue 205 (: labeling name (INCI: Blue 4)), Blue 404 (labeling name), Green 3 (: labeling name (INCI: Green 3, Green 3 Lake)), Green 201 (: labeling name (INCI: Green 5)), Green 202 (: labeling name (INCI: Green 6)), Green 204 (: labeling name (INCI: Green 8)), Green 205 (labeling name), Orange 201 (: labeling name (INCI: Orange 5)), Orange 203 (: labeling name (INCI: Pigment Orange 5)), Orange 204 (labeling name), Orange 205 (: labeling name (INCI: Orange 4, Orange 4 Lake)), Orange 206 (: labeling name (INCI: Orange 10)), and Orange 207 (: labeling name (INCI: Orange 11)); and natural dyes such as cochineal (INCI), laccaic acid (: labeling name (INCI: Laccaic Acid)), safflower Red (: labeling name (INCI: Carthamus Tinctorius (Safflower) Flower Extract)), purple root extract (: labeling name (INCI: Lithospermum Officinale Root Extract)), gardenia yellow (labeling name), and gardenia blue (: labeling name (INCI: Hydrolyzed Gardenia Florida Extract)).

· Inorganic-organic composite powder

Examples of the inorganic-organic composite powders include a composite powder in which the surface of an inorganic powder is coated with an organic powder by a known method.

In addition, for the above-described powders, the powder with particles surface-treated can be also used. In addition, from the viewpoint of water resistance of the cosmetic, it is preferable that the surface treatment agent is one that can impart hydrophobicity, and the treatment agent imparting hydrophobicity is not particularly limited, and examples thereof include silicone treatment agents, waxes, paraffins, organofluorine compounds including perfluoroalkyl and phosphate salts, surfactants, amino acids including N-acyl glutamic acid, and metal soaps such as aluminum stearate and magnesium myristate.

The treatment agent is more preferably a silicone treatment agent, and examples thereof include silanes or silylating agents such as triethoxycaprylylsilane (INCI) and trimethoxysilyldimethicone (INCI), dimethicone (INCI), methicone (INCI), hydrogen dimethicone (INCI), triethoxysilylethyl polydimethylsiloxyethyl dimethicone (INCI), triethoxysilylethyl polydimethylsiloxyethyl hexyl dimethicone (INCI), and (acrylates/tridecyl acrylate/triethoxysilylpropyl methacrylate/dimethicone methacrylate) copolymer (: labeling name (INCI): Acrylates/Tridecyl Acrylate/Triethoxysilylpropyl Methacrylate/Dimethicone Methacrylate Copolymer).

Specific examples of these silicone treatment agents include AES-3083, KF-99P, KF-9901, KF-9908, KF-9909, KP-574, and KP-541 manufactured by Shin-Etsu Chemical Co., Ltd.

Further, the above-described surface hydrophobic treatment agents may be used singly or in combination of two or more. Specific examples of the surface-treated colored pigments include KTP-09 series manufactured by Shin-Etsu Chemical Co., Ltd., particularly KTP-09W, 09R, 09Y, and 09B.

(5) Composition composed of crosslinked organopolysiloxane and oil agent that is liquid at room temperature

In a composition composed of a crosslinked organopolysiloxane and an oil agent that is liquid at room temperature, it is preferable that the crosslinked organopolysiloxane swells by including the liquid oil agent in an amount equal to or more than its own weight based on the liquid oil agent. As the above-described liquid oil agents, among the optional ingredients of (1) oil agent, liquid silicone oils, hydrocarbon oils, ester oils, natural animal and vegetable oils and fats, semi-synthetic oils and fats, fluorine-based oil agents, and the like can be used, and examples thereof include cyclopentasiloxane (INCI), dimethicone (INCI), mineral oil (INCI), isododecane (INCI), isohexadecane (INCI), triethylhexanoin (INCI), isotridecyl isononanoate (: labeling name (INCI: Isotridecyl isononanoate)), and squalane (INCI).

Unlike the crosslinked silicone surfactant of (3) ingredient as described above, (5) ingredient is a compound that does not have a polyether or polyglycerin structure in its molecular structure, and specific examples thereof include dimethicone/vinyl dimethicone crosspolymer (INCI), dimethicone/phenyl vinyl dimethicone crosspolymer (INCI), vinyl dimethicone/lauryl dimethicone crosspolymer (INCI), and lauryl polydimethylsiloxyethyl dimethicone/bis-vinyl dimethicone crosspolymer (INCI).

Examples of the composition composed of a commercially available crosslinked organopolysiloxane and an oil agent that is liquid at room temperature include KSG-15, KSG-1510, KSG-16, KSG-1610, KSG-19, KSG-016F, KSG-18A, KSG-41A, KSG-42A, KSG-43, KSG-44, KSG-042Z, KSG-045Z, and KSG-048Z manufactured by Shin-Etsu Chemical Co., Ltd.

(6) Film-forming agent

The film-forming agent is blended primarily for the purpose of further maintaining the durability of the effect of the cosmetic. There are no particular limitations, but a silicone-based composition is preferable from the viewpoint of imparting water repellency. Specifically, trimethylsiloxysilicate, acrylic-silicone film-forming agents, silicone-modified norbornene, silicone-modified pullulan, silicone-modified polyvinyl alcohol, and the like can be used.

Examples of the film-forming agents for silicone-based compositions include trimethylsiloxysilicate (: labeling name (INCI: Trimethylsiloxysilicate)), acrylates/dimethicone copolymer (INCI), norbornene/tris(trimethylsiloxy)silylnorbornene copolymer (INCI), and tri(trimethylsiloxy)silylpropylcarbamate pullulan (: labeling name (INCI: Trimethylsiloxysilylcarbamoyl Pullulan).

The above-described film-forming agent may be dissolved in a liquid oil agent at room temperature and then blended into the cosmetic. As the above-described liquid oil agents, among optional ingredients of (1) oil agent, liquid silicone oils, hydrocarbon oils, ester oils, natural animal and vegetable oils and fats, semi-synthetic oils and fats, fluorine-based oil agent, and the like can be used.

Specific examples of the commercially available film-forming agents for silicone-based compositions include KF-7312J, KP-545, KP-549, KP-543, NBN-30-ID, TSPL-30-ID, and TSPL-30-D5 manufactured by Shin-Etsu Chemical Co., Ltd.

(7) Ultraviolet absorbing and scattering agent

Examples of the ultraviolet absorbing and scattering agents include particles that absorb and scatter ultraviolet light, such as fine particle titanium dioxide, fine particle iron-containing titanium dioxide, fine particle zinc oxide, fine particle cerium oxide, and the composites thereof, and it is also possible to use a dispersion in which these particles that absorb and scatter ultraviolet light are previously dispersed in an oil agent.

As the oil agent, among optional ingredients of (1) oil agent, liquid silicone oils, hydrocarbon oils, ester oils, natural animal and vegetable oils and fats, semi-synthetic oils and fats, fluorine-based oil agents, and the like can be used.

Specific examples of the dispersions in which particles that absorb and scatter ultraviolet light are previously dispersed in an oil agent include SPD series (trade name) manufactured by Shin-Etsu Chemical Co., Ltd., in particular SPD-T5, T5L, Z5, Z5L, T6, Z6, T7, and Z7L.

(8) Other additives

Examples of the other additives include oil-soluble gelling agents, preservatives and bactericides, antiperspirants, fragrances, salts, antioxidants, pH adjusters, chelating agents, cooling agents, anti-inflammatory agents, skin-beautifying ingredients (whitening agents, cell activators, skin roughness-improving agents, blood circulation promoters, skin astringents, antiseborrheic agents, and the like), vitamins, amino acids, nucleic acids, hormones, and inclusion compounds.

· Oil-soluble gelling agent

Examples of the oil-soluble gelling agents include: metal soaps such as aluminum stearate, magnesium stearate, and zinc myristate; amino acid derivatives such as lauroyl glutamic acid (: labeling name (INCI: Lauroyl Glutamic Acid)) and $\alpha,\gamma$-di-n-butylamine; dextrin fatty acid esters such as dextrin palmitate (: labeling name (INCI: Dextrin Palmitate)), dextrin isostearate (: labeling name (INCI: Dextrin Isostearate)), dextrin myristate (: labeling name (INCI: Dextrin Myristate)), inulin stearate (: labeling name (INCI: Stearoyl Inulin)), and dextrin palmitate/ethylhexanoate (labeling name: Dextrin Palmitate/Ethylhexanoate)); sucrose fatty acid esters such as sucrose palmitate and sucrose stearate; fructooligosaccharide fatty acid esters such as fructooligosaccharide stearic acid esters and fructooligosaccharide 2-ethylhexanoic acid esters; benzylidene derivatives of sorbitol such as monobenzylidene sorbitol and dibenzylidene sorbitol; disteardimonium hectorite (INCI), stearalkonium hectorite (INCI), and organically modified clay minerals of hectorite; and stearalkonium bentonite (INCI).

· Preservative and bactericide

Examples of the preservatives and bactericides include alkyl parahydroxybenzoate, benzoic acid, sodium benzoate, sorbic acid, potassium sorbate, phenoxyethanol, imidazolidinyl urea, salicylic acid, isopropylmethylphenol, carbolic acid, parachlormetacresol, hexachlorophene, benzalkonium chloride, chlorhexidine chloride, trichlorocarbanilide, iodopropynyl butylcarbamate, polylysine, photosensitizer, silver, and plant extract.

· Antiperspirant

Examples of the antiperspirants include aluminum hydroxyhalides such as aluminum chlorohydrate, aluminum halides such as aluminum chloride, allantoin aluminum salt, tannic acid, persimmon tannin, (aluminum/potassium) sulfate, zinc oxide, zinc paraphenolsulfonate, burnt alum, tetrachloro(aluminum/zirconium) hydrate, and trichlorohydrex glycine (aluminum/zirconium). As particularly effective ingredients, aluminum hydroxyhalide, aluminum halide, complexes or mixtures of these with zirconyl oxyhalides and zirconyl hydroxyhalides (for example, tetrachloro(aluminum /zirconium) hydrate, trichlorohydrex glycine (aluminum /zirconium)), and the like are preferable.

· Fragrance

Examples of the fragrances include both natural fragrance and synthetic fragrance. The natural fragrances include: plant-based fragrances isolated from flowers, leaves, wood, and fruit peels; and animal-based fragrances such as musk and civet. Examples of the synthetic fragrances include: hydrocarbons such as monoterpenes; alcohols such as aliphatic alcohols and aromatic alcohols; aldehydes such as terpene aldehydes and aromatic aldehydes; ketones such as alicyclic ketones; esters such as terpene esters; lactones; phenols; oxides; nitrogen-containing compounds; and acetals.

· Salts

Examples of the salts include inorganic salts, organic acid salts, amine salts, and amino acid salts. Examples of the inorganic salts include sodium salts, potassium salts, magnesium salts, calcium salts, aluminum salts, zirconium salts, and zinc salts of inorganic acids such as hydrochloric acid, sulfuric acid, carbonic acid, and nitric acid; examples of the organic acid salts include organic acid salts such as acetic acid, dehydroacetic acid, citric acid, malic acid, succinic acid, ascorbic acid, and stearic acid; and examples of the amine salts and amino acid salts include salts of amines such as triethanolamine, and salts of amino acids such as glutamic acid. In addition, other salts such as salts of hyaluronic acid and chondroitin sulfate, aluminum zirconium glycine complex, and acid-alkali neutral salts used in cosmetic formulations can also be used.

· Antioxidant

The antioxidant is not particularly limited, and examples thereof include carotenoids, ascorbic acid and the salts thereof, ascorbyl stearate, tocopherol, tocopherol acetate, tocopherol, p-t-butylphenol, butylhydroxyanisole, dibutylhydroxytoluene, phytic acid, ferulic acid, thiotaurine, hypotaurine, sulfites, erythorbic acid and the salts thereof, chlorogenic acid, epicatechin, epigallocatechin, epigallocatechin gallate, apigenin, campherol, myricetin, and quercetin. The antioxidants may be used singly or in combination of two or more.

· pH adjuster

Examples of the pH adjusters include lactic acid, citric acid, glycolic acid, succinic acid, tartaric acid, dl-malic acid, potassium carbonate, sodium bicarbonate, and ammonium bicarbonate.

· Chelating agent

Examples of the chelating agents include alanine, edetate sodium salt, sodium polyphosphate, sodium metaphosphate, and phosphoric acid.

· Cooling agent

Examples of the cooling agents include L-menthol, camphor, and menthyl lactate.

· Anti-inflammatory agent

Examples of the anti-inflammatory agents include allantoin, glycyrrhizic acid and the salts thereof, glycyrrhetinic acid, stearyl glycyrrhetinate, tranexamic acid, and azulene.

· Skin-beautifying ingredient

Examples of the skin-beautifying ingredients include: whitening agents such as arbutin, glutathione, and saxifrage extract; wrinkle-improving agents such as retinol and niacinamide; cell activators such as royal jelly, photosensitizers, cholesterol derivatives, and calf blood extract; skin roughness-improving agents; blood circulation promoters such as nonyl acid valenylamide, nicotinic acid benzyl ester, nicotinic acid $\beta$-butoxyethyl ester, capsaicin, zingerone, cantharides tincture, ichthammol, caffeine, tannic acid, $\alpha$-borneol, tocopherol nicotinate, inositol hexanicotinate, cyclandelate, cinnarizine, tolazoline, acetylcholine, verapamil, cepharanthine, and $\gamma$-oryzanol; skin astringents such as zinc oxide and tannic acid; and antiseborrheic agents such as sulfur and thianthol.

· Vitamins

Examples of the vitamins include: vitamin A compounds such as vitamin A oil, retinol, retinol acetate, and retinol palmitate; vitamin B2 compounds such as riboflavin, riboflavin butyrate, and flavin adenine nucleotide; vitamin B6 compounds such as pyridoxine hydrochloride, pyridoxine dioctanoate, and pyridoxine tripalmitate; vitamin B compounds such as vitamin B12 and the derivatives thereof, vitamin B15 and the derivatives thereof; vitamin C compounds such as L-ascorbic acid, L-ascorbic acid dipalmitate, sodium L-ascorbic acid 2-sulfate, and L-ascorbic acid phosphate dipotassium; vitamin D compounds such as ergocalciferol and cholecalciferol; vitamin E compounds such as $\alpha$-tocopherol, $\beta$-tocopherol, $\gamma$-tocopherol, dl-$\alpha$-tocopherol acetate, dl-$\alpha$-tocopherol nicotinate, and dl-$\alpha$-tocopherol succinate; nicotinic acids such as nicotinic acid, benzyl nicotinate, and nicotinamide; vitamin H; vitamin P; pantothenic acids such as calcium pantothenate, D-pantothenyl alcohol, pantothenyl ethyl ether, and acetylpantothenyl ethyl ether; and biotin.

· Amino acids

Examples of the amino acids include glycine, valine, leucine, isoleucine, serine, threonine, phenylalanine, arginine, lysine, aspartic acid, glutamic acid, cystine, cysteine, methionine, and tryptophan.

· Nucleic acid

Examples of the nucleic acids include deoxyribonucleic acid.

· Hormone

Examples of the hormones include estradiol and ethenylestradiol.

· Inclusion compound

Examples of the inclusion compounds include cyclodextrin.

EXAMPLE

The present invention will be specifically described below using Examples, Comparative Examples, Synthesis Examples, and Comparative Synthesis Examples, but the present invention is not limited thereto.

[Synthesis Example 1]

20 g of Flamenco (registered trademark) Red (BASF SE), a pearl pigment with red interference light in specular reflection, 0.72 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and mixed uniformly for 30 minutes using a homomixer at 5,000 rpm; then 22.33 g of a 10% dispersion of spherical silica with an average particle size of 160 nm (monodispersity D90/D10 ≤ 3) was added and 6.0 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle ∆h between the interference color due to reflection of pearl pigment and the structural color due to reflection of spherical silica was 64.

[Synthesis Example 2]

20 g of Flamenco (registered trademark) Red (BASF SE), a pearl pigment with red interference light in specular reflection, 0.72 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 22.33 g of a 10% dispersion of spherical silica with an average particle size of 148 nm (monodispersity D90/D10 ≤ 3) was added and 6.0 g of 25% aqueous ammonia was added to make the system alkaline; 5.7 g of tetraethoxysilane as a binder was added dropwise over 1 hour under conditions of 40°C to 50°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle ∆h between the interference color due to reflection of pearl pigment and the structural color due to reflection of spherical silica was 66.

[Synthesis Example 3]

20 g of Flamenco (registered trademark) Red (BASF SE), a pearl pigment with red interference light in specular reflection, 0.72 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 33.5 g of a 10% dispersion of spherical silica with an average particle size of 304 nm (monodispersity D90/D10 = 1.97) was added and 6.0 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle ∆h between the interference color due to reflection of pearl pigment and the structural color due to reflection of spherical silica was 16.

[Synthesis Example 4]

20 g of Flamenco (registered trademark) Red (BASF SE), a pearl pigment with red interference light in specular reflection, 0.36 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 67.0 g of a 10% dispersion of spherical silica with an average particle size of 304 nm (monodispersity D90/D10 = 1.97) was added and 4.3 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle ∆h between the interference color due to reflection of pearl pigment and the structural color due to reflection of spherical silica was 16.

[Synthesis Example 5]

20 g of Flamenco (registered trademark) Red (BASF SE), a pearl pigment with red interference light in specular reflection, 0.36 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 100.0 g of a 10% dispersion of spherical silica with an average particle size of 490 nm (monodispersity D90/D10 ≤ 3) was added and 4.3 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle ∆h between the interference color due to reflection of pearl pigment and the structural color due to reflection of spherical silica was 54.

[Synthesis Example 6]

20 g of Flamenco (registered trademark) Red (BASF SE), a pearl pigment with red interference light in specular reflection, 0.36 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 22.33 g of a 10% dispersion of spherical silica with an average particle size of 82 nm (monodispersity D90/D10 ≤ 3) was added and 6.0 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle Δh between the interference color due to reflection of pearl pigment and the structural color due to reflection of spherical silica was 71.

[Synthesis Example 7]

20 g of Flamenco (registered trademark) Red (BASF SE), a pearl pigment with red interference light in specular reflection, 0.36 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 22.33 g of a 10% dispersion of spherical silica with an average particle size of 1,050 nm (monodispersity D90/D10 ≤ 3) was added and 6.0 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle Δh between the interference color due to reflection of pearl pigment and the structural color due to reflection of spherical silica was 86.

[Synthesis Example 8]

20 g of Flamenco (registered trademark) Red (BASF SE), a pearl pigment with red interference light in specular reflection, 0.36 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 5.6 g of a 10% dispersion of spherical silica with an average particle size of 304 nm (monodispersity D90/D10 = 1.97) was added and 6.0 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle Δh between the interference color due to reflection of pearl pigment and the structural color due to reflection of spherical silica was 16.

[Synthesis Example 9]

20 g of Flamenco (registered trademark) Red (BASF SE), a pearl pigment with red interference light in specular reflection, 0.36 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 111.1 g of a 10% dispersion of spherical silica with an average particle size of 800 nm (monodispersity D90/D10 ≤ 3) was added and 6.0 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle Δh between the interference color due to reflection of pearl pigment and the structural color due to reflection of spherical silica was 70.

[Synthesis Example 10]

20 g of Flamenco (registered trademark) Red (BASF SE), a pearl pigment with red interference light in specular reflection, 0.36 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 33.5 g of a 10% dispersion of spherical silica with an average particle size of 264 nm (monodispersity D90/D10 ≤ 3) was added and 6.0 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle Δh between the interference color due to reflection of pearl pigment and the structural color due to reflection of spherical silica was 172.

[Synthesis Example 11]

18 g of Flamenco (registered trademark) Red (BASF SE), a pearl pigment with red interference light in specular reflection, and 2 g of hydrophilic spherical silica with an average particle size of 304 nm (monodispersity D90/D10 = 1.97) were stirred in a sample mill (Kyoritsu Riko Co., Ltd.) for 1 minute three times in total, and the hydrophilic spherical silica was fixed by electrostatic adsorption to obtain a composite powder. The difference in hue angle Δh between the interference color due to reflection of pearl pigment and the structural color due to reflection of spherical silica was 16.

[Synthesis Example 12]

20 g of Flamenco (registered trademark) Red (BASF SE), a pearl pigment with red interference light in specular reflection, 0.36 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 33.5 g of a 10% dispersion of spherical silica with an average particle size of 304 nm (monodispersity D90/D10 = 1.97) was added and 6.0 g of 25% aqueous ammonia was added to make the system alkaline; 13 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle Δh between the interference color due to reflection of pearl pigment and the structural color due to reflection of spherical silica was 16.

[Synthesis Example 13]

20 g of Flamenco (registered trademark) Red (BASF SE), a pearl pigment with red interference light in specular reflection, 0.36 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 33.5 g of a 10% dispersion of spherical silica with an average particle size of 304 nm (monodispersity D90/D10 = 1.97) was added and 6.0 g of 25% aqueous ammonia was added to make the system alkaline; 0.5 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle Δh between the interference color due to reflection of pearl pigment and the structural color due to reflection of spherical silica was 16.

[Synthesis Example 14]

20 g of Flamenco (registered trademark) Red (BASF SE), a pearl pigment with red interference light in specular reflection, 0.72 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 22.33 g of a 10% dispersion of spherical silica with an average particle size of 148 nm (monodispersity D90/D10 ≤ 3) was added and 6.0 g of 25% aqueous ammonia was added to make the system alkaline; 5.7 g of tetraethoxysilane as a binder was added dropwise over 3 hour under conditions of 5°C to 10°C; then the mixture was aged for 1 hour, and then heated to 20°C to 30°C, and aged for 16 hours; and the mixture was filtered through filter paper (ADVANTEC CO., LTD., 5C) to obtain a composite powder. 5.75 g of this composite powder and 306 g of ion-exchanged water were placed in a 500 mL glass beaker and heated to 80°C, then 0.06 g of trimethylsilanol was added, the mixture was aged for 5 hours, and the mixture was filtered through filter paper (ADVANTEC CO., LTD., 5C) to obtain a composite powder. The difference in hue angle Δh between the interference color due to reflection of pearl pigment and the structural color due to reflection of spherical silica was 60.

[Synthesis Example 15]

20 g of Flamenco (registered trademark) Blue (BASF SE), a pearl pigment with blue interference light in specular reflection, 0.72 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and uniformly mixed with a homomixer at 5,000 rpm for 30 minutes; then 22.33 g of a 10% dispersion of spherical silica with an average particle size of 160 nm (monodispersity D90/D10 ≤ 3) was added and 6.0 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle Δh between the interference color due to reflection of pearl pigment and the structural color due to reflection of spherical silica was 1.6.

[Synthesis Example 16]

20 g of Flamenco (registered trademark) Green (BASF SE), a pearl pigment with green interference light in specular reflection, 0.72 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and uniformly mixed with a homomixer at 5,000 rpm for 30 minutes; then 22.33 g of a 10% dispersion of spherical silica with an average particle size of 180 nm (monodispersity D90/D10 $\leq$ 3) was added and 6.0 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder. The difference in hue angle ∆h between the interference color due to reflection of pearl pigment and the structural color due to reflection of spherical silica was 40.

[Comparative Synthesis Example 1]

Flamenco (registered trademark) Red (BASF SE), a pearl pigment with red interference light in specular reflection.

[Comparative Synthesis Example 2]

18 g of Flamenco (registered trademark) Red (BASF SE), a pearl pigment with red interference light in specular reflection, and 2 g of hydrophilic spherical silica with an average particle size of 304 nm (monodispersity D90/D10 = 1.97) were manually stirred to obtain a mixture. The obtained mixture was a simple, non-composite mixture.

[Comparative Synthesis Example 3]

20 g of Mearl Mica (BASF SE), 0.72 g of a cationic water-soluble polymer compound (product name: ME Polymer 09WPF, TOHO CHEMICAL INDUSTRY COMPANY, LIMITED), and 306 g of ion-exchanged water were placed in a 500 mL glass beaker and stirred for 30 minutes; then 33.5 g of a 10% dispersion of spherical silica with an average particle size of 304 nm (monodispersity D90/D10 = 1.97) was added and 0.6 g of 25% aqueous ammonia was added to make the system alkaline; 3.7 g of methyltrimethoxysilane as a binder was added dropwise over 1 hour under conditions of 5°C to 10°C; then the mixture was aged for 1 hour, and then heated to 50°C to 60°C, and aged for 1 hour; and the mixture was filtered through filter paper (ADVANTEC CO., LTD., 5C) to obtain a composite powder.

[Comparative Synthesis Example 4]

Flamenco (registered trademark) Blue (BASF SE), a pearl pigment with blue interference light in specular reflection.

[Comparative Synthesis Example 5]

18 g of Flamenco (registered trademark) Blue (BASF SE), a pearl pigment with blue interference light in specular reflection, and 2 g of hydrophobic spherical silica with an average particle size of 160 nm were manually stirred to obtain a mixture. The obtained mixture was a simple, non-composite mixture.

[Comparative Synthesis Example 6]

Flamenco (registered trademark) Green (BASF SE), a pearl pigment with green interference light in specular reflection.

[Comparative Synthesis Example 7]

18 g of Flamenco (registered trademark) Green (BASF SE), a pearl pigment with green interference light in specular reflection, and 2 g of hydrophobic spherical silica with an average particle size of 180 nm were manually stirred to obtain a mixture. The obtained mixture was a simple, non-composite mixture.

The following examples show the cases of using the obtained composite powder in cosmetics.

[Examples 1 to 14 and Comparative Examples 1 to 3]

Liquid emulsified cream

| Ingredient | | Mass (%) |
|---|---|---|
| 1. | KSG-210 (Note 1) | 3.5 |
| 2. | KSG-15 (Note 2) | 3 |
| 3. | KF-6017 (Note 3) | 0.5 |
| 4. | Dimethicone (6cs) | 5 |
| 5. | Cyclopentasiloxane | 6.5 |
| 6. | Powders of Synthesis Examples 1 to 14 and Comparative Synthesis Examples 1 to 3 | 5 |
| 7. | BG | 5.5 |
| 8. | Sodium citrate | 0.2 |
| 9. | Sodium chloride | 0.5 |
| 10. | Water | 70.3 |
| Total | | 100 |

(Note 1) A mixture of 70 to 80% dimethicone and 20 to 30% (dimethicone/(PEG-10/15)) crosspolymer, manufactured by Shin-Etsu Chemical Co., Ltd.
(Note 2) A mixture of 90 to 96% cyclopentasiloxane and 4 to 10% dimethicone/vinyl dimethicone crosspolymer, manufactured by Shin-Etsu Chemical Co., Ltd.
(Note 3) PEG-10 Dimethicone, manufactured by Shin-Etsu Chemical Co., Ltd.

(Production method)

Ingredients 1 to 6 were mixed in a beaker using a disperser, and separately mixed ingredients 7 to 10 were added at the same time. Further, these were thoroughly stirred and loaded into a container to obtain a liquid emulsified cream.

[Examples 15 and 16 and Comparative Examples 4 to 7]

Face powder

| Ingredient | | Mass (%) |
|---|---|---|
| 1. | Talc | 25 |
| 2. | Mica | 52 |
| 3. | Zinc stearate | 2 |
| 4. | Powders of Synthesis Examples 15 and 16 and Comparative Synthesis Examples 4 to 7 | 15 |
| 5. | Dimethicone (6cs) | 3 |
| 6. | Isotridecyl isononanoate | 3 |
| Total | | 100 |

(Production method)

Ingredients 1 to 4 were mixed in a Henschel mixer, and separately mixed ingredients 5 and 6 were added at the same time. Further, these were thoroughly mixed and press-molded to obtain a face powder.

(1) Usability evaluation

The liquid emulsified creams of Examples 1 to 14 and Comparative Examples 1 to 3 were evaluated for usability during application (light spreadability), concealment of blemishes and wrinkles after application, the lack of pearly gloss, and tone-up effect.
In addition, the face powders of Examples 15 and 16 and Comparative Examples 4 to 7 were evaluated for usability during application (light spreadability), concealment of blemishes and wrinkles after application, the lack of pearly gloss, and concealment of yellowish dullness and red face.

The evaluation was performed by 10 expert panelists. Evaluation was based on the evaluation criteria shown in Table 1, and the results were determined according to the following criteria based on the average of the 10 panelists' evaluations. The results for the liquid emulsified cream are shown in Table 2 below, and the results for the face powder are shown in Table 3 below.

[Table 1]

| Item | Degree of usability |
|---|---|
| Five points | Favorable |
| Four points | Slightly favorable |
| Three points | Normal |
| Two points | Slightly poor |
| One point | Poor |

(2) Usability criteria

A: Average score is 4.0 or more points
B: Average score is 3.0 or more and less than 4.0 points
C: Average score is 2.0 or more and less than 3.0 points
D: Average score is less than 2.0 points

A grade of C or more was considered a pass, and a grade of D in any item was considered a fail.

[Table 2]

| | Usability during application (Smoothness) | Wrinkles concealment (skin irregularities correction) | Blemish concealment (finish with transparency) | Lack of pearly gloss (suppression of unnatural luster) | Tone-up (brightening skin) |
|---|---|---|---|---|---|
| Example 1 | A | B | B | B | B |
| Example 2 | A | B | B | B | A |
| Example 3 | A | A | A | A | A |
| Example 4 | A | A | A | A | A |
| Example 5 | B | A | A | A | B |
| Example 6 | B | B | B | C | C |
| Example 7 | B | B | B | B | C |
| Example 8 | B | C | C | C | C |
| Example 9 | B | A | A | A | C |
| Example 10 | A | A | A | A | C |
| Example 11 | B | C | C | B | C |
| Example 12 | B | B | B | A | C |
| Example 13 | B | B | C | B | C |
| Example 14 | A | B | B | A | A |
| Comparative Example 1 | C | D | D | D | B |
| Comparative Example 2 | D | C | C | D | C |

(continued)

| | Usability during application (Smoothness) | Wrinkles concealment (skin irregularities correction) | Blemish concealment (finish with transparency) | Lack of pearly gloss (suppression of unnatural luster) | Tone-up (brightening skin) |
|---|---|---|---|---|---|
| Comparative Example 3 | B | B | B | B | D |

From the results in Table 2 above, the composite powder of the present invention used in the liquid emulsified creams of Examples 1 to 14 had favorable usability during application, and was confirmed to provide the effects of correcting skin irregularities, providing a transparent finish, suppressing pearly gloss, and brightening the skin. Among these, Examples 1 to 5 were particularly excellent for the effect of brightening the skin. In addition, it was confirmed that Example 14, which was hydrophobized, had the effect of suppressing pearly gloss compared to Example 2.

On the other hand, Comparative Examples 1 and 2, which do not combine pearl pigment and spherical silica, were inferior in usability during application, correction of skin irregularities, suppression of unnatural luster, and the effect of brightening the skin. Comparative Example 3, which used a powder different from the pearl pigment as its substrate, was effective in usability during application, correction of skin irregularities, transparent finish, and suppression of unnatural luster, but was inferior in the effect of brightening the skin.

[Table 3]

| | Usability during application (Smoothness) | Wrinkles concealment (skin irregularities correction) | Blemish concealment (finish with transparency) | Lack of pearly gloss (suppression of unnatural luster) | yellowish dullness concealment (brightening skin) |
|---|---|---|---|---|---|
| Example 15 | A | A | A | A | A |
| Example 16 | A | A | A | A | A |
| Comparative Example 4 | C | D | D | D | B |
| Comparative Example 5 | D | C | C | D | C |
| Comparative Example 6 | C | D | D | D | B |
| Comparative Example 7 | D | C | C | D | C |

From the results in Table 3 above, it was confirmed that the face powders of Examples 15 and 16 had favorable usability during application, and also had the effect of correcting skin irregularities, providing a transparent finish, and suppressing unnatural luster, and further, and by selecting the composite powder of the present invention, which exhibited an appropriate structural color, the effect of brightening the skin for skin color tone problems (yellowish dullness and red face) were provided.

On the other hand, Comparative Examples 4 to 7, which do not combine pearl pigment and spherical silica, were inferior in usability during application, correction of skin irregularities, transparent finish, suppression of unnatural luster, and effect of brightening the skin.

[Example 17]

Press foundation

| Ingredient | | Mass (%) |
|---|---|---|
| 1. | Liquid paraffin | 2 |
| 2. | Squalane | 2 |
| 3. | Dimethicone 20CS | 3 |
| 4. | Polyethylene | 5 |

EP 4 772 160 A1

(continued)

| Ingredient | | Mass (%) |
|---|---|---|
| 5. | KF-9901 (Note 1) treated sericite | 40 |
| 6. | Barium sulfate | 8 |
| 7. | KMP-590 (Note 2) | 5 |
| 8. | KF-9901 (Note 1) treated Talc | 15 |
| 9. | Composite powder from Synthesis Example 1 | 10 |
| 10. | KTP-09W (Note 3) | 9 |
| 11. | KTP-09R, Y, B (Note 3) | 1 |
| Total | | 100 |

(Note 1) Hydrogen Dimethicone, manufactured by Shin-Etsu Chemical Co., Ltd.
(Note 2) Polymethylsilsesquioxane, manufactured by Shin-Etsu Chemical Co., Ltd.
(Note 3) KF-9909 treated colored inorganic pigment, W: white, R: red, Y: yellow, B: black, manufactured by Shin-Etsu Chemical Co., Ltd.

(Production method)

A: Ingredients 4 to 11 were placed in a Henschel mixer and stirred and mixed.
B: Ingredients 1 to 3 were dissolved uniformly and mixed with A.
C: B was pulverized in a hammer mill, loaded into a mold and molded to obtain a press foundation.

The press foundation obtained as described above had a significantly favorable in-use feeling, and was confirmed to provide a natural, transparent finish while correcting skin irregularities, etc. and to have the effect of brightening the skin while suppressing unnatural luster.

[Example 18]

O/W Cream

| Ingredient | | Mass (%) |
|---|---|---|
| 1. | KSG-16 (Note 1) | 10 |
| 2. | Triethylhexanoin | 5 |
| 3. | Composite powder from Synthesis Example 2 | 1 |
| 4. | DPG | 7 |
| 5. | Glycerin | 5 |
| 6. | Metolose SM-4000 (2% aqueous solution) (Note 2) | 7 |
| 7. | Polyacrylamide emulsifier (Note 3) | 2 |
| 8. | Preservatives | Appropriate amount |
| 9. | Fragrances | Appropriate amount |
| 10. | Water | Balance |
| Total | | 100 |

(Note 1) A mixture of 70 to 80% dimethicone and 20 to 30% dimethicone/vinyl dimethicone crosspolymer, manufactured by Shin-Etsu Chemical Co., Ltd.
(Note 2) Methylcellulose manufactured by Shin-Etsu Chemical Co., Ltd.
(Note 3) Sepigel 305 (manufactured by SEPPIC S.A.)

(Production method)

27

A: Ingredients 4 to 10 were mixed.
B: Ingredients 1 to 3 were mixed and added to A, followed by stirring and emulsification.

The O/W cream obtained in this manner had a significantly favorable in-use feeling, and was confirmed to provide a natural, transparent finish while correcting skin irregularities, etc. and to have the effect of brightening the skin while suppressing unnatural luster.

[Example 19]

W/O Cream foundation

| Ingredient | | Mass (%) |
|---|---|---|
| 1. | KSG-310 (Note 1) | 2 |
| 2. | KSG-41A (Note 2) | 2 |
| 3. | Mineral oil | 2 |
| 4. | Triethylhexanoin | 5 |
| 5. | Isotridecyl isononanoate | 9 |
| 6. | KF-6038 (Note 3) | 1.5 |
| 7. | Composite powder from Synthesis Example 3 | 1 |
| 8. | KSP-100 (Note 4) | 2 |
| 9. | KTP-09W, R, Y, B (Note 5) | 10 |
| 10. | BG | 5 |
| 11. | Sodium citrate | 3 |
| 12. | Magnesium sulfate | 3 |
| 13. | Water | Balance |
| Total | | 100 |

(Note 1) A mixture of 65 to 75% mineral oil and 25 to 35% (PEG-15/lauryl dimethicone) crosspolymer, manufactured by Shin-Etsu Chemical Co., Ltd.
(Note 2) A mixture of 70 to 80% mineral oil and 20 to 30% vinyl dimethicone/lauryl dimethicone crosspolymer, manufactured by Shin-Etsu Chemical Co., Ltd.
(Note 3) Lauryl PEG-9 polydimethylsiloxyethyl dimethicone, manufactured by Shin-Etsu Chemical Co., Ltd.
(Note 4) Vinyl dimethicone/methicone silsesquioxane crosspolymer, manufactured by Shin-Etsu Chemical Co., Ltd.
(Note 5) KF-9909 treated colored inorganic pigment, W: white, R: red, Y: yellow, B: black, manufactured by Shin-Etsu Chemical Co., Ltd.

(Production method)

A: Ingredients 1 to 6 were mixed uniformly, and ingredients 7 to 9 were added and uniformed.
B: Ingredients 10 to 13 were mixed uniformly.
C: Under stirring, B was gradually added to A and emulsified.

The W/O cream foundation obtained in this manner had a significantly favorable in-use feeling, and was confirmed to provide a natural, transparent finish while correcting skin irregularities, etc. and to have the effect of brightening the skin while suppressing unnatural luster.

[Example 20]

W/O Cream

| Ingredient | | Mass (%) |
|---|---|---|
| 1. | KSG-310 (Note 1) | 6 |
| 2. | KSG-41A (Note 2) | 2 |
| 3. | Mineral oil | 13.5 |
| 4. | Macadamia nut oil | 5 |
| 5. | KF-6038 (Note 3) | 1 |
| 6. | KSP-100 (Note 4) | 2 |
| 7. | Composite powder from Synthesis Example 4 | 1 |
| 8. | Sodium citrate | 0.2 |
| 9. | DPG | 8 |
| 10. | Glycerin | 3 |
| 11. | Water | Balance |
| Total | | 100 |

(Note 1) A mixture of 65 to 75% mineral oil and 25 to 35% (PEG-15/lauryl dimethicone) crosspolymer, manufactured by Shin-Etsu Chemical Co., Ltd.
(Note 2) A mixture of 70 to 80% mineral oil and 20 to 30% vinyl dimethicone/lauryl dimethicone crosspolymer, manufactured by Shin-Etsu Chemical Co., Ltd.
(Note 3) Lauryl PEG-9 polydimethylsiloxyethyl dimethicone, manufactured by Shin-Etsu Chemical Co., Ltd.
(Note 4) Vinyl dimethicone/methicone silsesquioxane crosspolymer, manufactured by Shin-Etsu Chemical Co., Ltd.

(Production method)

A: Ingredients 1 to 7 were mixed uniformly.
B: Ingredients 8 to 11 were mixed uniformly.
C: Under stirring, B was gradually added to A and emulsified.

The W/O cream obtained in this manner had a significantly favorable in-use feeling, and was confirmed to provide a natural, transparent finish while correcting skin irregularities, etc. and to have the effect of brightening the skin while suppressing unnatural luster.

[Example 21]

W/O Cream

| Ingredient | | Mass (%) |
|---|---|---|
| 1. | Decamethylcyclopentasiloxane | 16 |
| 2. | Dimethicone 6CS | 4 |
| 3. | KF-6017 (Note 1) | 5 |
| 4. | Octyldodeceth-5 | 1 |
| 5. | Polysorbate 60 | 0.5 |
| 6. | Composite powder from Synthesis Example 5 | 2 |
| 7. | Plate barium sulfate | 2 |
| 8. | Mineral oil | 2 |
| 9. | Macadamia nut oil | 1 |
| 10. | Scutellaria root extract (Note 2) | 1 |
| 11. | Gentian extract (Note 3) | 0.5 |
| 12. | Ethanol | 5 |
| 13. | BG | 2 |
| 14. | Water | Balance |

(continued)

| Ingredient | Mass (%) |
|---|---|
| Total | 100 |

(Note 1) PEG-10 Dimethicone, manufactured by Shin-Etsu Chemical Co., Ltd.
(Note 2) 50% Scutellaria root extract, extracted with BG water
(Note 3) 20% Gentian extract, extracted with ethanol water

(Production method)

A: Ingredients 6 to 9 were mixed uniformly.
B: Ingredients 1 to 5 were mixed uniformly and A was added.
C: Ingredients 10 to 14 were mixed, and then under stirring, B was gradually added and emulsified.

The W/O cream obtained in this manner had a significantly favorable in-use feeling, and was confirmed to provide a natural, transparent finish while correcting skin irregularities, etc. and to have the effect of brightening the skin while suppressing unnatural luster.

[Example 22]

Eyeliner

| Ingredient | | Mass (%) |
|---|---|---|
| 1. | Decamethylcyclopentasiloxane | 39 |
| 2. | KF-6017 (Note 1) | 3 |
| 3. | KF-7312J (Note 2) | 15 |
| 4. | Disteardimonium hectorite | 3 |
| 5. | KF-9901 (Note 3) treated black iron oxide | 8 |
| 6. | Composite powder from Synthesis Example 15 | 2 |
| 7. | BG | 5 |
| 8. | Sodium dehydroacetate | appropriate amount |
| 9. | Preservative | appropriate amount |
| 10. | Water | Balance |
| Total | | 100 |

(Note 1) PEG-10 Dimethicone, manufactured by Shin-Etsu Chemical Co., Ltd.
(Note 2) 50% trimethylsiloxysilicate dissolved in cyclopentasiloxane, manufactured by Shin-Etsu Chemical Co., Ltd.
(Note 3) Hydrogen dimethicone, manufactured by Shin-Etsu Chemical Co., Ltd.

(Production method)

A: Ingredients 1 to 4 were mixed, and ingredients 5 and 6 were added and mixed and dispersed uniformly.
B: Ingredients 7 to 10 were mixed.
C: Under stirring, B was gradually added to A and emulsified.

The eyeliner obtained in this manner had a significantly favorable in-use feeling and provided a natural finish.

[Example 23]

Eye shadow

| Ingredient | | Mass (%) |
|---|---|---|
| 1. | Decamethylcyclopentasiloxane | 17 |
| 2. | Dimethicone 6cs | 10 |
| 3. | KF-6028 (Note 1) | 2 |
| 4. | Laureth-10 | 0.5 |
| 5. | Composite powder from Synthesis Example 16 | 9 |
| 6. | KF-9909 treated (Note 2) inorganic coloring material | appropriate amount |
| 7. | Sodium chloride | 2 |
| 8. | Propylene glycol | 8 |
| 9. | Preservative | appropriate amount |
| 10. | Water | Balance |
| Total | | 100 |

(Note 1) PEG-9 polydimethylsiloxyethyl dimethicone, manufactured by Shin-Etsu Chemical Co., Ltd.
(Note 2) Triethoxysilylethyl polydimethylsiloxyethyl hexyl dimethicone, manufactured by Shin-Etsu Chemical Co., Ltd.

(Production method)

A: Ingredients 1 to 4 were mixed, and ingredients 5 and 6 were added and mixed and dispersed uniformly.
B: Ingredients 7 to 10 were dissolved uniformly.
C: Under stirring, B was gradually added to A and emulsified.

The eye shadow obtained in this manner spread easily and was not oily or powdery, providing a soft in-use feeling. In addition, it was confirmed to have no unnatural luster and the effect of providing a natural, transparent finish.

[Example 24]

Lipstick

| Ingredient | | Mass (%) |
|---|---|---|
| 1. | Candelilla wax | 7 |
| 2. | Polyethylene wax | 8 |
| 3. | KP-561P (Note 1) | 12 |
| 4. | KF-54 (Note 2) | 3 |
| 5. | Isotridecyl isononanoate | 20 |
| 6. | Glyceryl isostearate | 16 |
| 7. | Polyglyceryl triisostearate | 28.5 |
| 8. | Composite powder from Synthesis Example 1 | 5.5 |
| 9. | Organic pigment | appropriate amount |
| Total | | 100 |

(Note 1) Acrylates/stearyl acrylate/dimethicone methacrylate copolymer, manufactured by Shin-Etsu Chemical Co., Ltd.
(Note 2) Diphenyl dimethicone, manufactured by Shin-Etsu Chemical Co., Ltd.

In the above ingredients, the total of ingredients 1 to 8 is 100% by mass due to rounding, but as described in the production method below, the lipstick of Example 24 includes ingredients 1 to 9.

(Production method)

A: Ingredients 1 to 6 and a part of ingredient 7 were heated, mixed, and dissolved.
B: Ingredients 8 and 9 and the remainder of ingredient 7 were mixed uniformly, and added to A and mixed uniformly.

The lipstick obtained in this manner spread easily and was not oily or powdery, providing a soft in-use feeling, and also had favorable water resistance and water repellency, lasted favorably, and was excellent in stability. It was confirmed to provide a natural, transparent finish and to have the effect of brightening the lips while suppressing unnatural luster.

[Example 25]

Lipstick

| Ingredient | | Mass (%) |
|---|---|---|
| 1. | Polyethylene | 7 |
| 2. | Microcrystalline wax | 3 |
| 3. | KP-561P (Note 1) | 10.5 |
| 4. | Triethylhexanoin | 19.5 |
| 5. | Neopentyl glycol diethylhexanoate | 15 |
| 6. | Neopentyl glycol dicaprate | 7 |
| 7. | Hydrogenated polyisobutene | 2.5 |
| 8. | KF-54HV (Note 2) | 7.5 |
| 9. | Diglyceryl triisostearate | 0.8 |
| 10. | Composite powder from Synthesis Example 14 | 6 |
| 11. | Colored pigment | 21.2 |
| Total | | 100 |

(Note 1): Acrylates/stearyl acrylate/dimethicone methacrylate copolymer, manufactured by Shin-Etsu Chemical Co., Ltd.
(Note 2) Diphenyl dimethicone, manufactured by Shin-Etsu Chemical Co., Ltd.

(Production method)

A: Ingredients 1 to 8 were heated, mixed, and dissolved.
B: Ingredients 9 and 11 were mixed uniformly and added to A and mixed uniformly.
C: Ingredient 10 was added to B and mixed uniformly.

The lipstick obtained in this manner spread easily and was not oily or powdery, providing a soft in-use feeling, and also had favorable water resistance and water repellency, lasted favorably, and was excellent in stability. It was confirmed to provide a natural, transparent finish and to have the effect of brightening the lips while suppressing unnatural luster.

[Example 26]

Nail enamel

| Ingredient | | Mass (%) |
|---|---|---|
| 1. | KP-543 (Note 1) | 45 |
| 2. | TMF-1.5 (Note 2) | 5 |

(continued)

| Ingredient | | Mass (%) |
|---|---|---|
| 3. | Nitrocellulose | 3 |
| 4. | Camphor | 0.5 |
| 5. | Acetyltributyl citrate | 1 |
| 6. | Dimethyldistearyl ammonium hectorite | 0.5 |
| 7. | Butyl acetate | 27 |
| 8. | Ethyl acetate | 10 |
| 9. | Isopropyl alcohol | 5 |
| 10. | Composite powder from Synthesis Example 3 | 3 |
| Total | | 100 |

(Note 1) 50% Acrylates/dimethicone copolymer dissolved in butyl acetate, manufactured by Shin-Etsu Chemical Co., Ltd.
(Note 2) Methyl trimethicone, manufactured by Shin-Etsu Chemical Co., Ltd.

(Production method)

A: Ingredients 7 to 9 were mixed, and then ingredients 4 to 6 were added and mixed uniformly.
B: Ingredients 1 to 3 were added to A and mixed.
C: Ingredient 10 was added to B and mixed.

The nail enamel obtained in this manner spread easily, provided a visually smooth appearance, was water-resistant and oil-resistant, lasted favorably, and was further confirmed to be excellent in stability with no pressure on the nails, no yellowing of the nails, and no changes in the cosmetic film due to temperature or aging.

[Example 27]

Cheek color

| Ingredient | | Mass (%) |
|---|---|---|
| 1. | KSG-16 (Note 1) | 28 |
| 2. | Decamethylcyclopentasiloxane | 34.5 |
| 3. | Neopentyl glycol dioctanoate | 9 |
| 4. | Stearoyl inulin | 10 |
| 5. | KSP-101 (Note 2) | 2 |
| 6. | Composite powder from Synthesis Example 14 | 16.5 |
| 7. | Red No. 202 | appropriate amount |
| 8. | KTP-09W, R, Y, B (Note 3) | appropriate amount |
| 9. | Tocopherol | appropriate amount |
| Total | | 100 |

(Note 1) A mixture of 70 to 80% dimethicone and 20 to 30% dimethicone/vinyl dimethicone crosspolymer, manufactured by Shin-Etsu Chemical Co., Ltd.
(Note 2) Vinyl dimethicone/methicone silsesquioxane crosspolymer, manufactured by Shin-Etsu Chemical Co., Ltd.
(Note 3) KF-9909 treated colored inorganic pigment, W: White, R: Red, Y: Yellow, B: Black, manufactured by Shin-Etsu Chemical Co., Ltd.

In the above ingredients, the total of ingredients 1 to 6 is 100% by mass due to rounding, but as described in the production method below, the cheek color of Example 27 includes ingredients 1 to 9.

(Production method)

A: Ingredients 1 to 5 were mixed, heated to 80° C, and uniformly dispersed.
B: Ingredients 6 to 9 were added to A, uniformly dispersed at 80°C, and then returned to room temperature.

The cheek color obtained in this manner had significantly favorable in-use feeling, and was confirmed to provide a natural, transparent finish while correcting skin irregularities, etc. and to have the effect of brightening the skin while suppressing unnatural luster.

[Example 28]

Eye color

| Ingredient | | Mass (%) |
|---|---|---|
| 1. | Isotridecyl isononanoate | 20 |
| 2. | Squalane | 20 |
| 3. | KSP-100 (Note 1) | 6 |
| 4. | Dextrin palmitate | 10 |
| 5. | KSG-16 (Note 2) | 12 |
| 6. | Barium sulfate | 5 |
| 7. | Polyethylene terephthalate/Aluminum powder | 4.5 |
| 8. | Titanium mica | 13.5 |
| 9. | Cosmetic glass flake powder (Note 3) | 1.5 |
| 10. | Composite powder from Synthesis Example 14 | 7.5 |
| 11. | Tocopherol | appropriate amount |
| Total | | 100 |

(Note 1) Vinyl dimethicone/methicone silsesquioxane crosspolymer, manufactured by Shin-Etsu Chemical Co., Ltd.
(Note 2) A mixture of 70 to 80% dimethicone and 20 to 30% dimethicone/vinyl dimethicone crosspolymer, manufactured by Shin-Etsu Chemical Co., Ltd.
(Note 3) Glass flakes (manufactured by Nippon Sheet Glass Co., Ltd.)

In the above ingredients, the total of ingredients 1 to 10 is 100% by mass due to rounding, but as described in the production method below, the eye color of Example 28 includes ingredients 1 to 11.

(Production method)

A: Ingredients 1 to 5 were mixed, heated to 90°C, and uniformly dispersed.
B: Ingredients 6 to 11 were added to A, and further uniformly dispersed at 90°C, then returned to room temperature.

The eye color obtained in this manner spread easily and was not oily or powdery, providing a soft in-use feeling. In addition, it was confirmed to have no unnatural luster and the effect of providing a natural, transparent finish.

[Example 29]

Cream lipstick

| Ingredient | | Mass (%) |
|---|---|---|
| 1. | dextrin palmitate/ethylhexanoate (Note 1) | 9 |
| 2. | Triethylhexanoin | 7 |
| 3. | KP-545 (Note 2) | 5 |
| 4. | KSG-43 (Note 3) | 8 |
| 5. | KF-6105 | 2 |
| 6. | Decamethylcyclopentasiloxane | 46 |
| 7. | BG | 5 |
| 8. | Water | 10.8 |
| 9. | Colorant | 1.2 |
| 10. | Composite powder from Synthesis Example 1 | 6 |
| Total | | 100 |

(Note 1) Rheopearl TT, manufactured by Chiba Flour Milling Co., Ltd.
(Note 2) Dissolved product of 70% cyclopentasiloxane and 30% acrylates/dimethicone copolymer, manufactured by Shin-Etsu Chemical Co., Ltd.
(Note 3) A mixture of 65 to 75% triethylhexanoin and 25 to 35% vinyl dimethicone/lauryl dimethicone crosspolymer, manufactured by Shin-Etsu Chemical Co., Ltd.

(Production method)

A: Ingredient 9 was mixed with a part of ingredient 2 and dispersed using a roller mill, the resulting dispersion was then heated and mixed with ingredient 1, and the remainder of ingredient 2, and ingredients 3 to 6.
B: Ingredients 7 and 8 were heated and added to the mixture obtained in A, followed by emulsification and cooling.
C: Ingredient 10 was added to the emulsion obtained in B to obtain a cream lipstick.

The resulting cream lipstick spread easily, was not sticky or oily, and formed a long-lasting film on the lips. It was confirmed to provide a natural, translucent finish and to have the effect of brightening lips while reducing unnatural luster.

[Example 30]

Water-in-oil foundation

| Composition | | Mass (%) |
|---|---|---|
| 1. | KSG-210 (Note 1) | 3.0 |
| 2. | KSG-15 (Note 2) | 2.0 |
| 3. | KF-6017 (Note 3) | 2.0 |
| 4. | Organically modified bentonite | 1.0 |
| 5. | Cyclopentasiloxane | 32.35 |
| 6. | KF-56A (Note 4) | 5.0 |
| 7. | Composite powder from Synthesis Example 4 | 2.0 |
| 8. | BG | 5.0 |
| 9. | NaCl | 0.5 |
| 10. | Sodium citrate | 0.2 |
| 11. | Water | 35.0 |
| 12. | Colored pigment paste | 11.95 |

(continued)

| Composition | Mass (%) |
|---|---|
| Total | 100.00 |

(Note 1) A mixture of 70 to 80% dimethicone and 20 to 30% (dimethicone/(PEG-10/15)) crosspolymer, manufactured by Shin-Etsu Chemical Co., Ltd.
(Note 2) A mixture of 90 to 96% cyclopentasiloxane and 4 to 10% dimethicone/vinyl dimethicone crosspolymer, manufactured by Shin-Etsu Chemical Co., Ltd.
(Note 3) PEG-10 dimethicone, manufactured by Shin-Etsu Chemical Co., Ltd.
(Note 4) Diphenylsiloxyphenyl trimethicone, manufactured by Shin-Etsu Chemical Co., Ltd.

(Production method)

A: Ingredients 1 to 7 were mixed uniformly.
B: Ingredients 8 to 11 were mixed uniformly.
C: B was added to A and emulsified.
D: Ingredient 12 was added to C and mixed uniformly.

The water-in-oil foundation obtained in this manner was a stable cosmetic. The water-in-oil foundation had a significantly favorable in-use feeling, and was confirmed to provide a natural, transparent finish while correcting skin irregularities, etc., and to have the effect of brightening the skin while suppressing unnatural luster.

[Example 31]

Water-in-oil water-break foundation

| Composition | | Mass (%) |
|---|---|---|
| 1. | KSG-710 (Note 1) | 3.0 |
| 2. | KSG-19 (Note 2) | 4.0 |
| 3. | KF-6104 (Note 3) | 0.3 |
| 4. | Composite powder from Synthesis Example 14 | 1.5 |
| 5. | KF-56A (Note 4) | 6.5 |
| 6. | Dimethicone 2cs | 3.0 |
| 7. | Dimethicone 6cs | 1.7 |
| 8. | Pentylene glycol | 5.0 |
| 9. | NaCl | 1.0 |
| 10. | Sodium citrate | 0.2 |
| 11. | Water | 66.5 |
| 12. | KF-6106 (Note 5) | 0.3 |
| 13. | KF-99P (Note 6) treated inorganic colored pigment | 7.0 |
| Total | | 100.0 |

(Note 1) A mixture of 70 to 80% by mass of dimethicone and 20 to 30% by mass of (dimethicone/polyglycerin-3) crosspolymer, manufactured by Shin-Etsu Chemical Co., Ltd.
(Note 2) A mixture of 80 to 90% by mass of dimethicone and 10 to 20% by mass of dimethicone/vinyl dimethicone crosspolymer, manufactured by Shin-Etsu Chemical Co., Ltd.
(Note 3) Polyglyceryl-3 polydimethylsiloxyethyl dimethicone, manufactured by Shin-Etsu Chemical Co., Ltd.
(Note 4) Diphenylsiloxyphenyl trimethicone, manufactured by Shin-Etsu Chemical Co., Ltd.
(Note 5) Polyglyceryl-3 polydimethylsiloxyethyl dimethicone, manufactured by Shin-Etsu Chemical Co., Ltd.
(Note 6) Methicone, manufactured by Shin-Etsu Chemical Co., Ltd.

(Production method)

A: Ingredients 1 to 6 and a part of ingredient 7 were mixed uniformly.
B: Ingredients 8 to 11 were mixed uniformly.
C: B was added to A and emulsified.
D: The remainder of ingredient 7, ingredients 12 and 13 were roll mixed, and then added to C and mixed uniformly.

The water-in-oil water-break foundation obtained in this manner was a stable cosmetic. The water-in-oil water-break foundation had a significantly favorable in-use feeling, and was confirmed to provide a natural, transparent finish while correcting skin irregularities, etc. and to have the effect of brightening the skin while suppressing unnatural luster.

[Example 32]

Oily mousse foundation

| Composition | | Mass (%) |
|---|---|---|
| 1. | Dimethicone (6cs) | Balance |
| 2. | KF-7312L (Note 1) | 10.0 |
| 3. | KSG-048Z (Note 2) | 30.0 |
| 4. | Squalane | 1.0 |
| 5. | Jojoba oil | 1.0 |
| 6. | KF-56A (Note 3) | 1.0 |
| 7. | Composite powder from Synthesis Example 14 | 18.0 |
| 8. | Methyl methacrylate crosspolymer | 1.0 |
| 9. | Nylon-12 | 1.0 |
| 10. | KTP-09W, R, Y, B (Note 4) | 10.0 |
| 11. | Stearic acid treated fine particle titanium dioxide | 10.0 |
| 12. | Antioxidant | appropriate amount |
| 13. | Dimethicone treated talc | 3.0 |
| 14. | Dimethicone treated mica | 5.0 |
| Total | | 100.0 |

(Note 1) Dissolved product of 50% by mass dimethicone (2cs) and 50% by mass trimethylsiloxysilicate, manufactured by Shin-Etsu Chemical Co., Ltd.
(Note 2) A mixture of 75 to 85% by mass dimethicone and 15 to 25% by mass lauryl polydimethylsiloxyethyl dimethicone/bisvinyl dimethicone crosspolymer, manufactured by Shin-Etsu Chemical Co., Ltd.
(Note 3) Diphenylsiloxyphenyl trimethicone, manufactured by Shin-Etsu Chemical Co., Ltd.
(Note 4) KF-9909 treated colored inorganic pigment, W: white, R: red, Y: yellow, B: black, manufactured by Shin-Etsu Chemical Co., Ltd.

(Production method)

A: A part of ingredient 1 and ingredients 2 to 9 were mixed uniformly.
B: Ingredients 10 to 14 are mixed with the remainder of ingredient 1 and roll processed.
C: The product obtained in step B was added to the product obtained in step A and mixed uniformly.
D: The product obtained in step C was degassed and then loaded into a container to obtain an oily mousse foundation.

The oily mousse foundation obtained in this manner had a mousse-like consistency that was easy to remove, spread easily, and had an in-use feeling that was neither oily nor powdery. In addition, the oily mousse foundation had a significantly favorable in-use feeling, and was confirmed to provide a natural, transparent finish while correcting skin irregularities, etc. and to have the effect of brightening the skin while suppressing unnatural luster.

As described above, the cosmetics blended with the composite powder of the present invention is a cosmetic that provides a natural, transparent finish while correcting skin irregularities, etc. and further provides the effect of brightening the skin while suppressing unnatural luster, and is excellent in in-use feeling such as smoothness.

The present description includes the following embodiments.

[1]: A composite powder, comprising a pearl pigment and spherical silica fixed thereon.

[2]: The composite powder of the above [1], wherein the spherical silica is in an amount of 10 to 50 parts by mass based on 100 parts by mass of the pearl pigment.

[3]: The composite powder of the above [1] or [2], wherein an average particle size of the spherical silica is in a range of 100 to 500 nm.

[4]: The composite powder of any one of the above [1] to [3], wherein a particle size distribution of the spherical silica is monodisperse.

[5]: The composite powder of any one of the above [1] to [4], wherein a hue angle difference $\Delta h$ between an interference color due to reflection of the pearl pigment and a structural color due to reflection of the spherical silica is in a range of 0 to 90.

[6]: The composite powder of any one of the above [1] to [5], wherein the spherical silica is chemically fixed on the pearl pigment with a binder.

[7]: The composite powder of the above [6], wherein the binder is a resin or silica.

[8]: The composite powder of the above [6] or [7], wherein the binder is in a range of 5 to 50 parts by mass based on 100 parts by mass of a total amount of the pearl pigment and the spherical silica.

[9]: The composite powder of any one of the above [1] to [8], wherein the composite powder is subjected to a hydrophobic surface treatment.

[10]: A cosmetic comprising the composite powder of any one of the above [1] to [9].

[11]: The cosmetic of the above [10], wherein the cosmetic is a skin care cosmetic.

[12]: The cosmetic of the above [10], wherein the cosmetic is a makeup cosmetic.

[13]: A composite powder, comprising a pearl pigment and a spherical powder fixed thereon.

It is noted that the present invention is not limited to the above-described embodiments. The above-described embodiments are merely examples, and any configuration that is substantially identical to the technical concept described in the claims of the present invention and that provides similar effects is within the technical scope of the present invention.

**Claims**

1. A composite powder, comprising a pearl pigment and spherical silica fixed thereon.

2. The composite powder according to claim 1, wherein the spherical silica is in an amount of 10 to 50 parts by mass based on 100 parts by mass of the pearl pigment.

3. The composite powder according to claim 1, wherein an average particle size of the spherical silica is in a range of 100 to 500 nm.

4. The composite powder according to claim 1, wherein a particle size distribution of the spherical silica is monodisperse.

5. The composite powder according to claim 1, wherein a hue angle difference $\Delta h$ between an interference color due to reflection of the pearl pigment and a structural color due to reflection of the spherical silica is in a range of 0 to 90.

6. The composite powder according to claim 1, wherein the spherical silica is chemically fixed on the pearl pigment with a binder.

7. The composite powder according to claim 6, wherein the binder is a resin or silica.

8. The composite powder according to claim 6, wherein the binder is in a range of 5 to 50 parts by mass based on 100 parts by mass of a total amount of the pearl pigment and the spherical silica.

9. The composite powder according to claim 1, wherein the composite powder is subjected to a hydrophobic surface treatment.

**10.** A cosmetic comprising the composite powder according to any one of claims 1 to 9.

**11.** The cosmetic according to claim 10, wherein the cosmetic is a skin care cosmetic.

**12.** The cosmetic according to claim 10, wherein the cosmetic is a makeup cosmetic.

**13.** A composite powder, comprising a pearl pigment and a spherical powder fixed thereon.

# EP 4 772 160 A1

## INTERNATIONAL SEARCH REPORT

| | International application No. |
|---|---|
| | **PCT/JP2024/029725** |

### A. CLASSIFICATION OF SUBJECT MATTER

*A61K 8/25*(2006.01)i; *A61Q 1/00*(2006.01)i; *A61Q 1/02*(2006.01)i; *A61Q 1/04*(2006.01)i; *A61Q 1/10*(2006.01)i; *A61Q 1/12*(2006.01)i; *A61Q 3/02*(2006.01)i; *A61Q 19/00*(2006.01)i; *C09C 3/06*(2006.01)i; *C09C 3/12*(2006.01)i
FI: A61K8/25; A61Q1/00; A61Q1/02; A61Q1/04; A61Q1/10; A61Q1/12; A61Q3/02; A61Q19/00; C09C3/06; C09C3/12

According to International Patent Classification (IPC) or to both national classification and IPC

### B. FIELDS SEARCHED

Minimum documentation searched (classification system followed by classification symbols)

A61K8/25; A61Q1/00; A61Q1/02; A61Q1/04; A61Q1/10; A61Q1/12; A61Q3/02; A61Q19/00; C09C3/06; C09C3/12

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
|---|---|---|
| X | JP 3-045660 A (MERCK PATENT GESELLSCHAFT MIT BESCHRAENKTER HAFTUNG) 27 February 1991 (1991-02-27) claims 1-7, examples 1, 3-16 | 1-5, 9-13 |
| Y | | 6-8, 10-12 |
| Y | WO 92/03119 A1 (CATALYSTS & CHEMICALS INDUSTRIES CO., LTD.) 05 March 1992 (1992-03-05) claims 1-8, p. 2, lines 10-16, p. 3, lines 5-10, examples 1-21 | 6-8, 10-12 |
| X | JP 2012-224587 A (NIPPON MENAADE KESHOHIN KK) 15 November 2012 (2012-11-15) claims 1-8 | 13 |
| X | JP 9-048707 A (SHISEIDO COMPANY, LTD.) 18 February 1997 (1997-02-18) claims 1-4 | 13 |
| A | JP 2001-98186 A (MERCK JAPAN KK) 10 April 2001 (2001-04-10) claims 1-6, examples 1-2 | 1-13 |

[✓] Further documents are listed in the continuation of Box C.    [✓] See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **11 October 2024** | **29 October 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

## INTERNATIONAL SEARCH REPORT

| International application No. |
| --- |
| **PCT/JP2024/029725** |

**C.      DOCUMENTS CONSIDERED TO BE RELEVANT**

| Category* | Citation of document, with indication, where appropriate, of the relevant passages | Relevant to claim No. |
| --- | --- | --- |
| A | JP 2007-291066 A (KOSE CORPORATION) 08 November 2007 (2007-11-08) claims 1-4, paragraph [0023], examples 1-4, 8, table 1 | 1-13 |

Form PCT/ISA/210 (second sheet) (July 2022)

## INTERNATIONAL SEARCH REPORT
### Information on patent family members

International application No.

**PCT/JP2024/029725**

| Patent document cited in search report | | | Publication date (day/month/year) | Patent family member(s) | Publication date (day/month/year) |
|---|---|---|---|---|---|
| JP | 3-045660 | A | 27 February 1991 | US 5407746 A<br>claims 1-7, examples 1, 3-16<br>EP 406657 A1 | |
| WO | 92/03119 | A1 | 05 March 1992 | US 2002/0011186 A1<br>claims 1-8, paragraphs [0011],<br>[0015], examples 1-21<br>EP 543999 A1 | |
| JP | 2012-224587 | A | 15 November 2012 | (Family: none) | |
| JP | 9-048707 | A | 18 February 1997 | (Family: none) | |
| JP | 2001-98186 | A | 10 April 2001 | US 6432535 B1<br>claims 1-6, examples 1-2<br>EP 1072651 A1<br>CN 1282766 A | |
| JP | 2007-291066 | A | 08 November 2007 | (Family: none) | |

Form PCT/ISA/210 (patent family annex) (July 2022)

**EP 4 772 160 A1**

**REFERENCES CITED IN THE DESCRIPTION**

**Patent documents cited in the description**

- JP 2005097218 A
- JP 2784261 B
- JP 5359593 B